(19) Europäisches Patentamt
European Patent Office
Office européen des brevets

(11) EP 1 585 728 B1

(12) EUROPÄISCHE PATENTSCHRIFT

(45) Veröffentlichungstag und Bekanntmachung des Hinweises auf die Patenterteilung:
**24.02.2010 Patentblatt 2010/08**

(21) Anmeldenummer: **03814463.0**

(22) Anmeldetag: **19.12.2003**

(51) Int Cl.:
*C07D 209/42* $^{(2006.01)}$ *C07D 209/52* $^{(2006.01)}$
*C07D 409/12* $^{(2006.01)}$ *C07D 417/14* $^{(2006.01)}$

(86) Internationale Anmeldenummer:
**PCT/EP2003/014611**

(87) Internationale Veröffentlichungsnummer:
**WO 2004/060874 (22.07.2004 Gazette 2004/30)**

# (54) IMINOSÄUREDERIVATE ALS INHIBITOREN VON MATRIX-METALLOPROTEINASEN

IMINO ACID DERIVATIVES FOR USE AS INHIBITORS OF MATRIX METALLOPROTEINASES

DERIVES D'AMINOACIDES UTILISES EN TANT QU'INHIBITEURS DE METALLOPROTEINASES MATRICIELLES

(84) Benannte Vertragsstaaten:
**AT BE BG CH CY CZ DE DK EE ES FI FR GB GR HU IE IT LI LU MC NL PT RO SE SI SK TR**

(30) Priorität: **03.01.2003 DE 10300015**

(43) Veröffentlichungstag der Anmeldung:
**19.10.2005 Patentblatt 2005/42**

(73) Patentinhaber: **Sanofi-Aventis Deutschland GmbH**
**65929 Frankfurt am Main (DE)**

(72) Erfinder:
- **SCHUDOK, Manfred**
  **65817 Eppstein/Ts. (DE)**
- **RUF, Sven**
  **65926 Frankfurt am Main (DE)**
- **MATTER, Hans**
  **63505 Langenselbold (DE)**
- **WEHNER, Volkmar**
  **97657 Sandberg (DE)**
- **KIRSCH, Reinhard**
  **38100 Braunschweig (DE)**
- **STAHL, Petra**
  **65926 Frankfurt am Main (DE)**

(56) Entgegenhaltungen:
**EP-A- 0 769 498** **WO-A-00/58304**
**WO-A-01/62733** **WO-A-97/18194**
**US-A1- 2003 008 861**

- **HODGSON, DAVID M. ET AL: "Development of dirhodium(II)-catalyzed generation and enantioselective 1,3-dipolar cycloaddition of carbonyl ylides" CHEMISTRY--A EUROPEAN JOURNAL , 7(20), 4465-4476 CODEN: CEUJED; ISSN: 0947-6539, 2001, XP002275259**
- **NUHRICH, A. ET AL: "Cyclization of N-tosyloxiranylpropylamines. Synthesis of nitrogen heterocycles" TETRAHEDRON , 47 (18-19), 3075-88 CODEN: TETRAB; ISSN: 0040-4020, 1991, XP002275260**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 585 728 B1

**Beschreibung**

Die Erfindung betrifft neue Derivate der Octahydroindolcarbonsäure sowie der Octahydrocyclopenta[b]pyrrolo-2-carbonsäure, Verfahren zu ihrer Herstellung und Verwendung derselben als Arzneimittel.

In Erkrankungen wie Osteoarthritis und Rheuma findet eine Zerstörung des Gelenkes statt, besonders bedingt durch den proteolytischen Abbau von Kollagen durch Kollagenasen. Kollagenasen gehören zur Superfamilie der Metalloproteinasen (MP) bzw. Matrix-Metallproteinasen (MMP's). Die MMP's bilden eine Gruppe von Zn-abhängigen Enzymen, die am biologischen Abbau der extrazellulären Matrix beteiligt sind (D. Yip et al. in Investigational New Drugs 17 (1999), 387-399 und Michaelides et al. in Current Pharmaceutical Design 5 (1999) 787 -819). Diese MMP's sind insbesondere fähig, fibrilläres und nicht-fibrilläres Kollagen, sowie Proteoglycane abzubauen, die beide wichtige Matrixbestandteite darstellen. MMP's sind beteiligt an Prozessen der Wundheilung, der Tumorinvasion, Metastasenwanderung sowie an Angiogenese, multipler Sklerose und Herzversagen (Michaelides Seite 788; siehe oben). Insbesondere spielen sie eine wichtige Rolle beim Abbau der Gelenkmatrix in der Arthrose und der Arthritis, sei es nun die Osteoarthrose, Osteoarthritis oder die rheumatoide Arthritis.

Die Aktivität der MMP's ist weiterhin essentiell für viele der Prozesse, die bei der atherosklerotischen Plaque-Bildung eine Rolle spielen, wie die Infiltration inflammatorischer Zellen, der glatten Muskelzell-Migration sowie Proliferation und Angiogenese (S. J. George, Exp. Opin. Invest. Drugs (2000), 9 (5), 993-1007.). Weiterhin kann die Matrix-Degradation durch MMP's Plaque-Instabilitäten bis hin zu Rupturen verursachen, was zu den klinischen Symptomen der Atherosklerose, instabilen Angina Pectoris, Myokardinfarkt oder Schlaganfall führen kann (E. J. M. Creemers et al, Circulation Res. 89, 201-210 (2001)). Insgesamt betrachtet kann die gesamte MMP-Familie alle Komponenten der extrazellulären Matrix der Blutgefäße abbauen; deshalb ist deren Aktivität in starkem Maße Regulationsmechanismen in normalen Blutgefäßen unterworfen. Die erhöhte MMP-Aktivität während der Plaque-Bildung und Plaque-Instabilität wird durch erhöhte Cytokin- und Growth-Faktor-stimulierte Gen-Transkription, erhöhte Zymogen-Aktivivierung und eine Ungleichgewicht des MMP-TIMP-Verhältnisses verursacht (Tissue inhibitors of metalloproteases). Deshalb erscheint es einleuchtend, dass eine MMP-Inhibierung oder die Wiedererlangung der MMP-TIMP-Gleichgewicht hilfreich sein wird in der Behandlung der atherosklerotischen Erkrankungen. Ebenso wird es immer deutlicher, dass neben der Atherosklerose auch weitere cardiovaskuläre Erkrankungen durch eine erhöhte MMP-Aktivität zumindest mitverursacht werden, wie beispielsweise Restenose, dilatierte Cardiomyopathie und der schon erwähnte Myokardinfarkt. Es konnte gezeigt werden, dass durch die Applikation synthetischer Inhibitoren in experimentellen Tiermodellen dieser Erkrankungen deutliche Verbesserungen erzielt werden konnten, z. B. betreffend Bildung atherosklerotischer Läsionen, Neointima-Bildung, Linksventrikuläres Remodelling, Dysfunktion der Pumpleistung oder Infarkt-Heilung. In verschiedenen präklinischen Studien mit MMP-Inhibitoren zeigte sich bei detaillierter Gewebsanalyser eine reduzierte Kollagen-Schädigung, verbessertes extrazelluläres Matrix-Remodelling und verbesserte Struktur und Funktion von Herzmuskel und Gefäßen. Von diesen Prozessen werden insbesondere die Matrix-Remodelling-Prozesse und MMP-regulierte Fibrosen als wichtige Komponenten im Fortschreiten von Herzerkrankungen (Infarkt) angesehen (Drugs 61,1239-1252 (2001)).

MMP's spalten Matrixproteine wie Kollagen, Laminin, Proteoglykane, Elastin oder Gelatin sowie prozessieren (d. h. aktivieren oder deaktivieren) durch eine Spaltung eine Vielzahl weiterer Proteine und Enzyme unter physiologischen Bedingungen, so dass Ihnen eine wichtige Rolle im gesamten Organismus zukommt, mit besonderer Bedeutung im Bindegewebe und Knochen.

Eine Vielzahl von verschiedenen Inhibitoren der MMP's sind bekannt (EP 0 606 046; WO 94/28889; WO 96/27583; oder auch Übersichten wie Current Medicinal Chemistry 8, 425-74 (2001). Nach den ersten klinischen Studien an Menschen hat sich nun gezeigt, dass MMP's Nebenwirkungen hervorrufen. Die hauptsächlich genannten Nebenwirkungen sind muskuloskeletale Schmerzen oder Anthralgien. Der Stand der Technik besagt eindeutig, dass erwartet wird, dass selektivere Inhibitoren diese genannten Nebenwirkungen reduzieren könne (Yip, Seite 387, siehe oben). Besonders hervorzuheben ist dabei eine Spezifität gegenüber MMP-1, da mit der Hemmung von MMP-1 offensichtlich diese unerwünschten Nebenwirkungen verstärkt auftreten.

Nachteil der bekannten Inhibitoren der MMP's sind daher häufig die mangelnde Spezifität. Die meisten MMP-Inhibitoren hemmen viele MMP's gleichzeitig, weil die katalytische Domäne der MMP's eine ähnliche Struktur aufweist. Demzufolge wirken die Inhibitoren in unerwünschter Weise auf die Enzyme, auch solche mit vitaler Funktion, ein (Massova I, et al., The FASEB Journal (1998) 12, 1075-1095).

In dem Bestreben, wirksame Verbindungen zur Behandlung von Bindegewebserkrankungen zu finden, wurde nun gefunden, dass die erfindungsgemäß eingesetzten Derivate starke Inhibitoren der Matrix-Metalloproteinasen MMP-2, MMP-3 MMP-8, MMP-9 und MMP-13 sind, während nur eine schwache Hemmung der MMP-1.

Die Erfindung betrifft daher eine Verbindung der Formel I

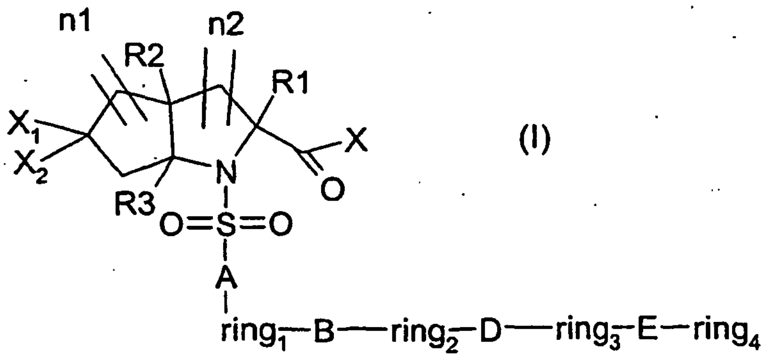

(I)

und/oder alle stereoisomeren Formen der Verbindung der Formel I und/oder Gemische diese Formen in jedem Verhältnis, und/oder ein physiologisch verträgliches Salz der Verbindung der Formel I, wobei

A für -($C_0$-$C_4$)-Alkylen steht,
B, D und E gleich oder verschieden sind und unabhängig voneinander für

1. -($C_0$-$C_4$)-Alkylen,
2. -($C_2$-$C_4$)-Alkenylen,
3. -$S(O)_o$-, wobei o die ganzen Zahlen Null, I oder 2 bedeutet,
4. -NH-,
5. -NH-C(O)-,
6. -C(O)-NH-,
7. -NH-$SO_2$-,
8. -NH-C(O)-NH-,
9. -NH-C(S)-,
10. -NH-C(O)-O-,
11. -O-,
12. -O-C(O)-NH-,
13. -C(O)-,
14. -O-$(CH_2)_n$-O-, worin n die ganze Zahl 2 oder 3 bedeutet, oder
15. -O-$(CH_2)_m$-NH-, worin m die ganze Zahl 2 oder 3 bedeutet, stehen,

ring1, ring2 oder ring3 gleich oder verschieden sind und unabhängig voneinander für

1. kovalente Bindung,
2. -($C_6$-$C_{14}$)-Aryl, worin Aryl unsubstituiert oder unabhängig voneinander ein-, zwei- oder dreifach durch G substituiert ist, oder
3. 5- oder 6-gliedriger aromatischer Heteroarylring stehen, worin der Heteroarylring unsubstituiert oder unabhängig voneinander ein-, zwei- oder dreifach durch G substituiert ist,

ring4 für

1. -($C_6$-$C_{14}$)-Aryl, worin Aryl unsubstituiert oder unabhängig voneinander ein-, zwei- oder dreifach durch G substituiert ist,
2. 5- oder 6-gliedriger aromatischer Heteroarylring, worin der Heteroarylring unsubstituiert oder unabhängig voneinander ein-, zwei- oder dreifach durch G substituiert ist,
3. Heteroaryl, worin Heteroaryl unsubstituiert oder unabhängig voneinander ein-, zwei- oder dreifach durch G substituiert ist, oder
4. für einen der folgenden Reste

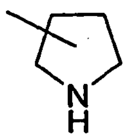 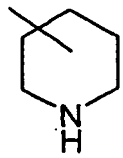 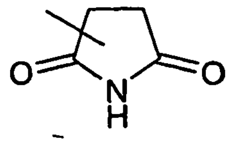 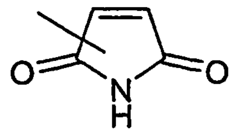 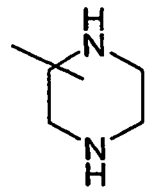 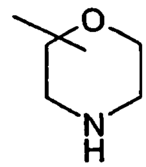

und diese Reste unsubstituiert oder unabhängig voneinander ein-, zwei- oder dreifach durch G substituiert sind, steht,

G für

1. Wasserstoffatom,
2. Halogen,
3. R4 steht und R4

   a) Wasserstoffatom,
   b) -($C_1$-$C_6$)-Alkyl, worin Alkyl unsubstituiert oder ein-, zwei- oder dreifach durch Halogen, -($C_3$-$C_6$)-Cycloalkyl, -($C_6$-$C_{14}$)-Aryl oder Heteroaryl substituiert ist,
   c) -($C_6$-$C_{14}$)-Aryl,
   d) Heteroaryl,
   e) -C(O)-O-R5, worin R5

      e)1) -($C_1$-$C_6$)-Alkyl, worin Alkyl unsubstituiert oder ein- oder zweifach durch -($C_3$-$C_6$)-Cycloalkyl, -($C_6$-$C_{14}$)-Aryl, oder Heteroaryl substituiert ist, oder
      e)2) -($C_6$-$C_{14}$)-Aryl oder Heteroaryl bedeutet,

   f) -C(S)-O-R5, worin R5 wie oben definiert ist,
   g) -C(O)-NH-R6, worin R6

      g)1) -($C_1$-$C_6$)-Alkyl, worin Alkyl unsubstituiert oder ein- oder zweifach durch -($C_3$-$C_6$)-Cycloalkyl, -($C_6$-$C_{14}$)-Aryl oder Heteroaryl substituiert ist, oder
      g)2) -($C_6$-$C_{14}$)-Ary) oder Heteroaryl bedeutet, oder

   h) -C(S)-NH-R6, worin R6 wie oben definiert ist, bedeutet,

4. -0-R4, worin R4 wie oben definiert ist,
5. -C(O)-R5, worin R5 wie oben definiert ist,
6. -$S(O)_p$-R4, worin R4 wie oben definiert ist und p die ganzen Zahlen Null, 1 oder 2 bedeutet,
7. -$NO_2$,
8. -CN oder
9. -N(R3)-R4, worin R3

   9.1) Wasserstoffatom oder
   9.2) -($C_1$-$C_6$)-Alkyl bedeutet und R4 wie oben definiert ist, steht,

X für -OHoder -NH-OH steht,
$X_1$ und $X_2$ gleich oder verschieden sind und unabhängig voneinander Wasserstoffatom oder -($C_1$-$C_6$)-Alkyl bedeuten, oder zusammen den Rest =O bilden,
n1 und n2 gleich oder verschieden sind und unabhängig voneinander Null, 1 oder 2 bedeuten,
R1 für

1. Wasserstoffatom oder
2. -($C_1$-$C_6$)-Alkyl, worin Alkyl unsubstituiert oder ein- oder zweifach durch -($C_3$-$C_6$)-Cycloalkyl, -($C_6$-$C_{14}$)-Aryl, oder Heteroaryl substituiert ist, steht,

R2 und R3 gleich oder verschieden sind und unabhängig voneinander für Wasserstoffatom oder -($C_1$-$C_6$)-Alkyl stehen,

wobei die Verbindung 1-(Toluol-4-sulfonyl)-octahydro-indol-2-carbonsäure (Tetrahedron Vol. 47, Nr. 18-19, Seite 3077) ausgeschlossen ist.

Ein weiterer Gegenstand der Erfindung ist die Verbindung der Formel I, wobei

A für -($C_0$-$C_4$)-Alkylen steht,
B, D und E gleich oder verschieden sind und unabhängig voneinander für

1. -($C_0$-$C_4$)-Alkylen,
2. -($C_2$-$C_4$)-Alkenylen,
3. -$S(O)_o$-, wobei o die ganzen Zahlen Null, 1 oder 2 bedeutet,
4. -NH-,
5. -NH-C(O)-,
6. -C(O)-NH-,
7. -NH-$SO_2$-,
8. -NH-C(O)-NH-,
9. -NH-C(S)-,
10. -NH-C(O)-O-,
11. -O-,
12. -O-C(O)-NH-,
13. -C(O)-,
14. -O-$(CH_2)_n$-O-, worin n die ganze Zahl 2 oder 3 bedeutet, oder
15. -O-$(CH_2)_m$-NH-, worin m die ganze Zahl 2 oder 3 bedeutet, stehen,

ring1, ring2 oder ring3 gleich oder verschieden sind und unabhängig voneinander für

1. kovalente Bindung,
2. -($C_6$-$C_{14}$)-Aryl, worin Aryl ein Rest aus der Reihe Phenyl, Naphthyl, 1-Naphthyl, 2-Naphthyl, Biphenylyl, 2-Biphenylyl, 3-Biphenylyl, 4-Biphenylyl, Anthryl oder Fluorenyl bedeutet und unsubstituiert oder unabhängig voneinander ein-, zwei- oder dreifach durch G substituiert sind, stehen, oder
3. 5- oder 6-gliedriger aromatischer Heteroarylring stehen, worin der Heteroarylring ein Rest aus der Reihe Dihydrofuranyl, Dioxolyl, Dioxanyl, Furanyl, Imidazolidinyl, Imidazolinyl, Imidazolyl, Isoxazolyl, Isoxazolidinyl, 2-Isoxazolinyl, Isothiazolyl, Isothiazolidinyl, 2-Isothiazolinyl, Morpholinyl, Oxazolyl, Oxothiolanyl, Piperazinyl, Piperidinyl, Pyranyl, Pyrazinyl, Pyrazolyl, Pyrazolidinyl, Pyrazolinyl, Pyridazinyl, Pyridinyl, Pyrimidinyl, Pyrrolyl, Pyrrolidinyl, Tetrahydrofuranyl, Tetrahydropyridinyl, Thiazolyl, Thiomorpholinyl, Thiophenyl oder Thiopyranyl bedeutet und unsubstituiert oder unabhängig voneinander ein-, zwei- oder dreifach durch G substituiert sind,

ring4 für

1. -($C_6$-$C_{14}$)-Aryl steht, worin Aryl ein Rest aus der Reihe Phenyl, Naphthyl, 1-Naphthyl, 2-Naphthyl, Biphenylyl, 2-Biphenylyl, 3-Biphenylyl, 4-Biphenylyl, Anthryl oder Fluorenyl bedeutet und unsubstituiert oder unabhängig voneinander ein-, zwei- oder dreifach durch G substituiert sind,
2. 5- oder 6-gliedriger aromatischer Heteroarylring steht, worin der Heteroarylring ein Rest aus der Reihe Dihydrofuranyl, Dioxolyl, Dioxanyl, Furanyl, Imidazolidinyl, Imidazolinyl, Imidazolyl, Isoxazolyl, Isoxazolidinyl, 2-Isoxazolinyl, Isothiazolyyl, Isothiazolidinyl, 2-Isothiazolinyl, Morpholinyl, Oxazolyl, Oxothiolanyl, Piperazinyl, Piperidinyl, Pyranyl, Pyrazinyl, Pyrazolyl, Pyrazolidinyl, Pyrazolinyl, Pyridazinyl, Pyridinyl, Pyrimidinyl, Pyrrolyl, Pyrrolidinyl, Tetrahydrofuranyl, Tetrahydropyridinyl, Thiazolyl, Thiomorpholinyl, Thiophenyl oder Thiopyranyl bedeutet und unsubstituiert oder unabhängig voneinander ein-, zwei- oder dreifach durch G substituiert sind,
3. Heteroary steht, worin Heteroaryl ein Rest aus der Reihe Acridinyl, Azetidinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chromanyl, Chromenyl, Cinnolinyl, Deca-hydrochinolinyl, 2H, 6H-1,5,2-Dithiazinyl, Dihydrofuran[2,3-b]-tetrahydrofuran, Fuaranyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazoly!, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridinyl, Pyridyl, Pyrimidinyl, Pyrrolidinyl, Pyrrolinyl, 2H-Pyrrolyl, Pyrrolyl, Chinazolinyl, Chinolinyl, 4H-Chinolizinyl, Chinoxalinyl, Chinuclidinyl, Tetrahydrofuranyl, Tetrahydroisochinolinyl, Tetrahydrochinolinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Thiophenyl, Triazinyl,1,2,3-Triazolyl, 1,2,3-Triazolyl, 1,2,4-Triazolyl, 1,2,5-Triazolyl, 1,3,4-Triazolyl und Xanthenyl bedeutet und unsubstituiert oder unabhängig voneinander ein-, zwei- oder dreifach durch G substituiert sind, oder
4. für einen der folgenden Reste

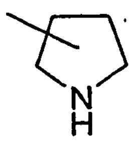 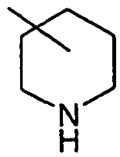 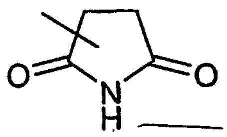 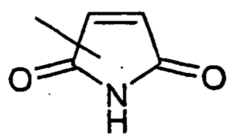 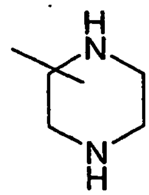 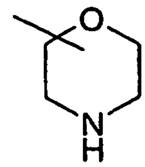

und diese Reste unsubstituiert oder unabhängig voneinander ein-, zwei- oder dreifach durch G substituiert sind, steht,

G für

1. Wasserstoffatom,
2. Halogen,
3. R4 steht und R4

a) Wasserstoffatom,
b) -($C_1$-$C_6$)-Alkyl, worin Alkyl unsubstituiert oder ein-, zwei- oder dreifach durch Halogen, -($C_3$-$C_6$)-Cycloalkyl, Phenyl, Naphthyl oder Heteroaryl, wobei Heteroaryl wie oben definiert, substituiert ist,
e) Phenyl oder Naphthyl,
f) Heteroaryl, wobei Heteroaryl wie oben definiert ist,
e) -C(O)-O-R5, worin R5

e)1) -($C_1$-$C_6$)-Alkyl, worin Alkyl unsubstituiert oder ein- oder zweifach durch -($C_3$-$C_6$)-Cycloalkyl, Phenyl, Naphthyl oder Heteroaryl, wobei Heteroaryl wie oben definiert ist, substituiert ist,
e)2) Phenyl oder Naphthyl oder
e)3) Heteroaryl, wobei Heteroaryl wie oben definiert, substituiert ist, bedeutet,

f) -C(S)-O-R5, worin R5 wie oben definiert ist,
g) -C(O)-NH-R6, worin R6

g)1) -($C_1$-$C_6$)-Alkyl, worin Alkyl unsubstituiert oder ein- oder zweifach durch -($C_3$-$C_6$)-Cycloalkyl, phenyl, Naphthyl oder Heteroaryl, wobei Heteroaryl wie oben definiert ist, substituiert ist,
g)2) Phenyl oder Naphthyl oder
g)3) Heteroaryl, wobei Heteroaryl wie oben definiert, substituiert ist, bedeutet, oder

h) -C(S)-NH-R6, worin R6 wie oben definiert ist, bedeutet,

4. -0-R4, worin R4 wie oben definiert ist,
5. -C(O)-R5, worin R5 wie oben definiert ist,
6. -S(O)p-R4, worin R4 wie oben definiert ist und p die ganzen Zahlen Null, 1 oder 2 bedeutet,
7. -$NO_2$,
8. -CN oder
9. -N(R3)-R4, worin R3

9.1) Wasserstoffatom oder

9.2) -($C_1$-$C_6$)-Alkyl bedeutet und R4 wie oben definiert ist, steht,

X für -OH oder -NH-OH steht,
$X_1$ und $X_2$ gleich oder verschieden sind und unabhängig voneinander Wasserstoffatom oder -($C_1$-$C_6$)-Alkyl bedeuten, oder zusammen den Rest =O bilden,
n1 und n2 gleich oder verschieden sind und unabhängig voneinander Null, 1 oder 2 bedeuten,
R1 für

1. Wasserstoffatom oder
2. -($C_1$-$C_6$)-Alkyl, worin Alkyl unsubstituiert oder ein- oder zweifach durch -($C_3$-$C_6$)-Cycloalkyl, Phenyl, Naphthyl oder Heteroaryl, wobei Heteroaryl wie oben definiert ist, substituiert ist, steht,

R2 und R3 gleich oder verschieden sind und unabhängig voneinander für Wasserstoffatom oder -($C_1$-$C_6$)-Alkyl stehen,

wobei die Verbindung 1-(Toluol-4-sulfonyl)-octahydro-indol-2-carbonsäure (Tetrahedron Vol. 47, Nr. 18-19, Seite 3077) ausgeschlossen ist.

Ein weiterer Gegenstand der Erfindung ist die Verbindung der Formel I, wobei

A für -($C_0$-$C_4$)-Alkylen steht,
B, D und E gleich oder verschieden sind und unabhängig voneinander für

1. -($C_0$-$C_2$)-Alkylen,
2. -$C_2$-Alkenylen,
3. -$S(O)_o$-, wobei o die ganzen Zahl 2 bedeutet,
4. -NH-,
5. -NH-C(O)-,
6. -C(O)-NH-,
7. -NH-C(O)-NH-,
8. -O- oder
9. -C(O)-, stehen,

ring1, ring2 oder ring3 gleich oder verschieden sind und unabhängig voneinander für

1. kovalente Bindung stehen,
2. Phenyl oder Naphthyl bedeutet und unsubstituiert oder unabhängig voneinander ein-, zwei- oder dreifach durch G substituiert sind, oder
3. 5- oder 6-gliedriger aromatischer Heteroarylring stehen, worin der Heteroarylring ein Rest aus der Reihe Dihydrofuranyl, Furanyl, Morpholinyl, Piperazinyl, Piperidinyl, Pyridinyl, Pyrimidinyl, Pyrrolyl, Thiazolyl oder Thiophenyl bedeutet und unsubstituiert oder unabhängig voneinander ein-, zwei- oder dreifach durch G substituiert sind,

ring4 für

1. Phenyl oder Naphthyl steht und unsubstituiert oder unabhängig voneinander ein-, zwei- oder dreifach durch G substituiert ist,
2. 5- oder 6-gliedriger aromatischer Heteroarylring steht, worin der Heteroarylring ein Rest aus der Reihe Dihydrofuranyl, Furanyl, Morpholinyl, Piperazinyl, Piperidinyl, Pyridinyl, Pyrimidinyl, Pyrrolyl, Thiazolyl oder Thiophenyl bedeutet und bedeutet und unsubstituiert oder unabhängig voneinander ein-, zwei- oder dreifach durch G substituiert ist,
3. Heteroaryl steht, worin Heteroaryl ein Rest aus der Reihe Benzofuranyl, Benzothiophenyl, Dihydrofuranyl, Furanyl, Morpholinyl, Piperazinyl, Piperidinyl, Pyridinyl, Pyridothiophenyl, Pyrimidinyl, Pyrrolyl, Thiazolyl oder Thiophenyl bedeutet und unsubstituiert oder unabhängig voneinander ein-, zwei- oder dreifach durch G substituiert sind, oder
4. für einen der folgenden Reste

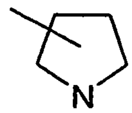   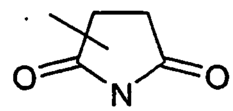

und diese Reste unsubstituiert oder unabhängig voneinander ein-, zwei- oder dreifach durch G substituiert sind, steht,

G für

1. Wasserstoffatom,
2. Br; Cl, I oder F,
3. R4 steht und R4

   a) Wasserstoffatom,
   b) -($C_1$-$C_4$)-Alkyl, worin Alkyl unsubstituiert oder ein-, zwei- oder dreifach durch Br, Cl, F, -$C_3$-Cycloalkyl, Phenyl, Naphthyl oder Heteroaryl, wobei Heteroaryl wie oben definiert, substituiert ist,
   g) Phenyl oder Naphthyl,
   h) Heteroaryl, wobei Heteroaryl wie oben definiert ist,
   e) -C(O)-O-R5, worin R5

      e)1) -($C_1$-$C_4$)-Alkyl, worin Alkyl unsubstituiert oder ein- oder zweifach durch -$C_3$-Cycloalkyl, Phenyl, Naphthyl oder Heteroaryl, wobei Heteroaryl wie oben definiert ist, substituiert ist,
      e)2) Phenyl oder Naphthyl oder
      e)3) Heteroaryl, wobei Heteroaryl wie oben definiert, substituiert ist, bedeutet, oder

   f) -C(O)-NH-R6, worin R6 für

      f)1) -($C_1$-$C_4$)-Alkyl, worin Alkyl unsubstituiert oder ein- oder zweifach durch -$C_3$-Cycloalkyl, Phenyl, Naphthyl oder Heteroaryl, wobei Heteroaryl wie oben definiert ist, substituiert ist,
      f)2) Phenyl oder Naphthyl oder
      f)3) Heteroaryl, wobei Heteroaryl wie oben definiert, substituiert ist, steht, bedeutet,

4. -O-R4, worin R4 wie oben definiert ist,
5. -C(0)-R5, worin R5 wie oben definiert ist,
6. -S(0)p-R4, worin R4 wie oben definiert ist und p die ganzen Zahl 2 bedeutet,
7. -$NO_2$,
8. -CN oder
9. -N(R3)-R4, worin R3

   9.1) Wasserstoffatom oder
   9.2) -($C_1$-$C_6$)-Alkyl bedeutet und R4 wie oben definiert ist, steht,

X für -OH oder-NH-OH steht,
$X_1$ und $X_2$ gleich sind und Wasserstoffatom bedeuten,
h1 und n2 gleich sind und 1 bedeuten, oder ungleich sind und n1 gleich 2 und n2 gleich 1 bedeuten,
R1 für Wasserstoffatom steht, und
R2 und R3 gleich sind und für Wasserstoffatom stehen. ,

Unter dem Begriff "($C_1$-$C_6$)-Alkyl" werden Kohlenwasserstoffreste verstanden, deren Kohlenstoffkette geradkettig oder verzweigt ist und 1 bis 6 Kohlenstoffatome enthält, beispielsweise Methyl, Ethyl, Propyl, Iso-Propyl, Butyl, Iso-Butyl, tertiär-Butyl, Pentyl, IsoPentyl, Neopentyl, Hexyl, 2,3-Dimethylbutan oder Neohexyl. Unter dem Begriff "-($C_0$-$C_4$)-Alkylen" werden Kohlenwasserstoffreste verstanden, deren Kohlenstoffkette geradkettig oder verzweigt ist und 1 bis 4 Kohlenstoffatome enthält, beispielsweise Methylen, Ethylen, Propylen, Iso-Propylen, Iso-Butylen, Butylen oder tertiär-Butylen. "-$C_0$-Alkylen" ist eine kovalente Bindung.
Unter dem Begriff "-$(CH_2)_n$-, worin n die ganze Zahl Null, I oder 2 bedeutet" wird für n gleich Null eine kovalente Bindung, n gleich 1 der Rest Methylen und n gleich 2 der Rest Ethylen verstanden. Unter dem Begriff "-($C_2$-$C_4$)-Alkenylen," werden

Kohlenwasserstoffreste verstanden, deren Kohlenstoffkette geradkettig oder verzweigt ist und 2 bis 4 Kohlenstoffatome enthält und je nach Kettenlänge,1 oder 2 Doppelbindungen aufweisen, beispielsweise Ethenylen, Propenylen, Iso-Propenylen, Iso-Butenylen oder Butenylen; die Substituenten an der Doppelbindung können, sofern die prinzipielle Möglichkeit besteht, E- oder Z-ständig angeordnet sein.
Unter dem Begriff "-($C_2$-$C_6$)-Alkinylen," werden Kohlenwasserstoffreste verstanden, deren Kohlenstoffkette geradkettig oder verzweigt ist und 2 bis 6 Kohlenstoffatome enthält und je nach Kettenlänge 1 oder 2 Dreifachbindüngen aufweisen, beispielsweise Ethinylen, Propenylen, Iso-Propinylen, Iso-Buthylinylen, Butinylen, Pentinylen oder Isomere von Pentinylen oder Hexinylen oder Isomere von Hexinylen.
Unter dem Begriff "($C_3$-$C_6$)-Cycloalkyl" werden Reste verstanden wie Verbindungen, die sich von 3- bis 6-gliedrige Monocyclen wie Cyclopropyl, Cyclobutyl, Cyclopentyl oder Cyclohexyl herleiten.
Unter den Resten

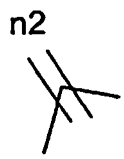

oder

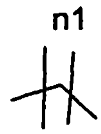

werden jeweils -$CH_2$-Reste im Ring der Formel I verstanden, wobei die Variablen n1 oder n2 jeweils die Anzahl der -$CH_2$-Reste im Ring der Formel I angeben. Für den Fall, dass n1 den Wert Null hat, ergibt sich eine kovalente Bindung und der erhaltene Teilring hat insgesamt 4 Ringatome. Für den Fall, dass n2 den Wert Null hat, ergibt sich eine kovalente Bindung und der erhaltene Teilring hat insgesamt 3 Ringatome. Für den Fall, dass n1 den Wert 1 hat, ergibt sich ein -$CH_2$-Rest und der erhaltene Teilring hat insgesamt 5 Ringatome. Für den Fall, dass n1 den Wert 2 hat, ergibt sich ein -$CH_2$-$CH_2$-Rest und der erhaltene Teilring hat insgesamt 6 Ringatome. Für den Fall, dass n1 den Wert 3 hat, ergibt sich ein -$CH_2$-$CH_2$-$CH_2$-Rest und der erhaltene Teilring hat insgesamt 7 Ringatome. Für n2 ergeben sich entsprechende Teilringe.
Unter dem Begriff "-($C_6$-$C_{14}$)-Aryl" werden aromatische Kohlenstoffreste verstanden mit 6 bis 14 Kohlenstoffatomen im Ring. -($C_6$-$C_{14}$)-Arylreste sind beispielsweise Phenyl, Naphthyl, zum Beispiel 1-Naphthyl, 2-Naphthyl, Anthryl oder Fluorenyl. Naphthylreste und insbesondere Phenylreste sind bevorzugte Arylreste.
Unter dem Begriff "5- oder 6-gliedriger aromatischer Heteroarylring" werden aromatische Ringsysteme verstanden, die ein oder zwei Heteroatome aus der Reihe Sauerstoff, Stickstoff oder Schwefel enthalten und sich von Dihydrofuran, Dioxol, Dioxan, Furan, Imidazolidin, Imidazolin, Imidazol, Isoxazol, Isoxazolidin, 2-Isoxazolin, Isothiazol, Isothiazolidin, 2-Isothiazolin, Morpholin, Oxazol, Oxothiolan, Piperazin, Piperidin, Pyran, Pyrazin, Pyrazol, Pyrazolidin, Pyrazolin, Pyridazin, Pyridin, Pyrimidin, Pyrrol, Pyrrolidin, Tetrahydrofuran, Tetrahydropyridin, Thiazol, Thiomorpholin, Thiophenyl oder Thiopyran ableiten lassen.
Unter dem Begriff "Heteroaryl" werden Reste wie Acridinyl, Azepinyl, Azetidinyl, Aziridinyl, Benzimidazalinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Oxothiolanyl, Pyrimidinyl; Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyi, 1,2,4+Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Thiomorpholinyl, Thiophenyl, Triazinyl, 1,2,3-Triazolyl, 1,2,3-Triazolyl, 1,2,4-Triazolyl, 1,2,5-Triazolyl, 1,3,4-Triazolyl und Xanthenyl verstanden. Bevorzugt sind Pyridyl; wie 2-Pyridyl, 3-Pyridyl oder 4-Pyridyl; Pyrrolyl; wie 2-Pyrrolyl und 3-Pyrrolyl; Furyl; wie 2-Furyl und 3-Furyl; Thiophenyl, Thienyl; wie 2-Thienyl und 3-Thienyl; Imidazolyl, Pyrazolyl, Oxazolyl, Isoxazolyl, Thiazolyl, Isothiazolyl, Tetrazolyl, Pyridazinyl, Pyrazinyl, Pyrimidinyl, Indolyl, Isoindolyl, Benzofuranyl, Benzothiophenyl, 1,3-Benzodioxolyl, Indazolyl, Benzimidazolyl, Benzoxazolyl, Benzothiazolyl, Chinolinyl, Isochinolinyl, Chromanyl, Isochromanyl, Cinnolinyl, Chinazolinyl, Chinoxalinyl, Phthalazinyl, Pyridoimidazolyl, Pyridopyridinyl, Pyridopyrimidinyl, Purinyl und Pteridinyl.

Die Erfindung betrifft ferner ein Verfahren zur Herstellung der Verbindung der Formel I und/oder einer stereoisomeren Form der Verbindung der Formel I und/oder eines physiologisch verträglichen Salzes der Verbindung der Formel I, das dadurch gekennzeichnet ist, dass man

a) eine Verbindung der Formel IV,

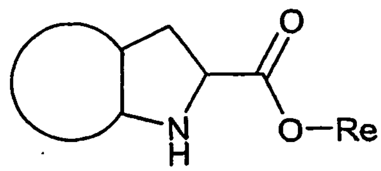

(IV)

worin Re ein Wasserstoffatom oder eine Ester-Schutzgruppe darstellt, mit einer Verbindung der Formel V,

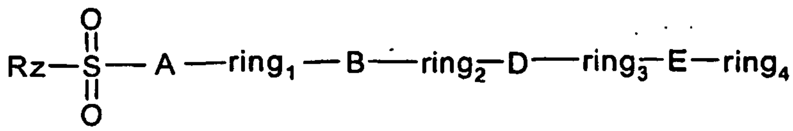

(V)

worin A, B, D, E und ring1, ring2, ring3, ring4 wie in Formel I definiert sind, und worin Rz Chloratom, Imidazoyl oder OH bedeutet, in Gegenwart einer Base oder nach Silylierung mit einem geeigneten Silylierungsmittel zu einer Verbindung der Formel VI umsetzt,

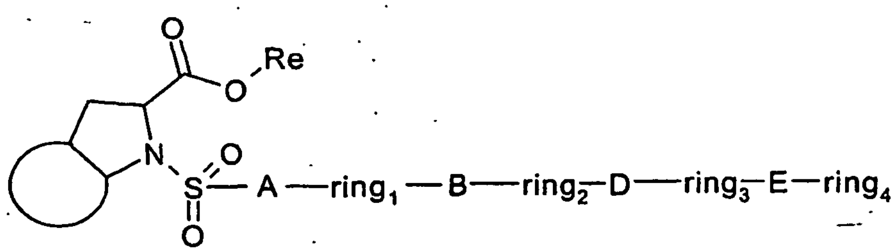

(VI)

worin A, B, D, E, Re und ring1, ring2, ring3 und ring4 wie oben definiert sind, und

b) für den Fall Re = Ester eine nach a) hergestellte Verbindung der Formel VI mit einer Alkalilauge wie NaOH oder LiOH und anschließender Säurebehandlung zu der Carbonsäure der Formel I, worin X = OH (entspricht Formel Vii), umsetzt, wobei gegebenenfalls vorher noch Modifikationen in einer der Seitenketten der Ringe ring1-ring4 vorgenommen wurden

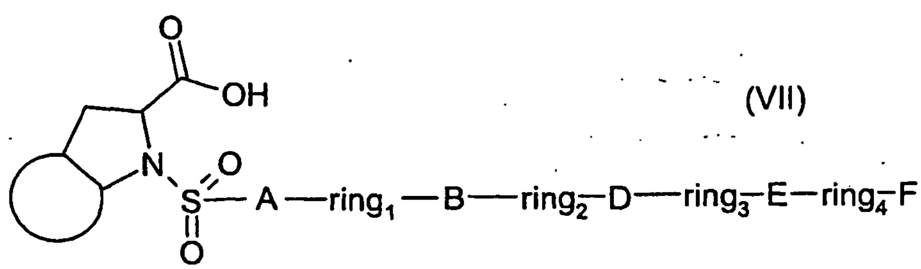

(VII)

und anschließend diese in die erfindungsgemäße Hydroxamsäure X = NH-OH der Formel I umwandelt,

c) eine nach Verfahren a) oder b) hergestellte Verbindung der Formel I, die aufgrund ihrer chemischen Struktur in enantiomeren Formen auftritt, durch Salzbildung mit enantiomerenreinen Säuren oder Basen, Chromatographie an

chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder

d) die nach den Verfahren b) oder c) hergestellte Verbindung der Formel I entweder in freier Form isoliert oder im Falle des Vorliegens von sauren oder basischen Gruppen in physiologisch verträgliche Salze umwandelt.

Verbindungen wie sie die Formeln IV bis VII stellen nur beispielhafte Verbindungen dar, anstatt des Fünfringes können entsprechend der Formel I auch Vierringe, Sechsringe und Siebenringe aufgeführt werden.

Als Ester-Schutzgruppe Re können die in Protective Groups in Organic Synthesis, T.H. Greene, P. G. M. Wuts, Wiley-Interscience, 1991, als Schutzgruppen für Ester verwendete Gruppen eingesetzt werden. Bevorzugte Ester-Schutzgruppen sind beispielsweise Methyl, Ethyl, Isopropyl, tert. Butyl oder Benzyl.
Die eingesetzten Ausgangsprodukte und Reagenzien können entweder nach bekannten Verfahren hergestellt werden oder sind käuflich erhältlich.
Die Umsetzungen erfolgen beispielsweise wie in WO 97/18194 dargestellt. Die Umsetzung gemäß Verfahrensschritt a) erfolgt in Gegenwart einer Base wie KOH, NaOH, LiOH, N,O-Bis-(trimethylsilyl)acetamid (BSA), N-Methylmorpholin (NMM), N-Ethylmorpholin (NEM), Triethylamin (TEA), Diisopropylethylamin (DIPEA), Pyridin, Collidin, Imidazol oder Natriumcarbonat in Lösungsmitteln wie Tetrahydrofuran (THF), Dimethylformamid (DMF), Dimethylacetamid, Dioxan, Acetonitril, Toluol, Chloroform oder Methylenchlorid, oder auch in Gegenwart von Wasser.

Modifikationen in der Seitenkette F bedeutet, dass beispielsweise eine Nitrogruppe mit dem Metallkatal,ysator Pd/C hydriert oder mit $SnCl_2$ bzw. Zn unter Standardbedingungen umgesetzt wird und die erhaltene Aminogruppe anschließend weiter modifiziert werden kann, beispielsweise durch Umsetzung mit Carbonsäurechloriden, Sulfonsäurechloriden, Chlorameisensäureestern, Isocyanaten, Isothiocyanaten oder anderen reaktiven oder aktivierbaren Reagenzien, um zu den Vorstufen der erfindungsgemäßen Verbindungen der Formel zu gelangen. Für diesen Fall ist es oft günstig, dass Re in Verbindung VI ein Ester ist, da im Falle der ungeschützten Carbonsäure mit Nebenreaktionen zu rechnen ist.

Im Verfahrensschritt-c) wird die Verbindung der Formel I, sofern sie als Gemisch von Diastereomeren oder Enantiomeren auftritt oder bei der gewählten Synthese als deren Gemische anfällt, in die reinen Stereoisomeren getrennt, entweder durch Chromatographie an einem gegebenenfalls chiralen Trägermaterial, oder, sofern die racemischen Verbindung der Formel I zur Salzbildung befähigt ist, durch fraktionierte Kristallisation der mit einer optisch aktiven Base oder Säure als Hilfsstoff gebildeten diastereomeren Salze. Als chirale Stationärphasen für die dünnschicht- oder säulenchromatographische Trennung von Enantiomeren eignen sich zum Beispiel modifizierte Kieselgelträger (sogenannte Pirkle-Phasen) sowie hochmolekulare Kohlenhydrate wie Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren werden mit einer optisch aktiven, in der Regel kommerziell erhältlichen Base wie (-)-Nicotin, (+)- und (-)-Phenyl-ethylamin, Chininbasen, L-Lysin oder L-und D-Arginin die unterschiedlich löslichen diastereomeren Salze gebildet, die schwerer lösliche Komponente als Feststoff isoliert, das leichter lösliche Diastereomer aus der Mutterlauge abgeschieden und aus den so gewonnenen diastereomeren Salzen die reinen Enantiomeren gewonnen. Auf prinzipiell gleiche Weise kann man die racemischen Verbindungen der Formel I, die eine basische Gruppe wie eine Aminogruppe enthalten, mit optisch aktiven Säuren, wie (+)-Campher-10-sulfonsäure, D- und L- Weinsäure, D-und L- Milchsäure sowie (+) und (-)-Mandelsäure in die reinen Enantiomeren überführen. Auch kann man chirale Verbindungen, die Alkohol- oder Amin-funktionen enthalten, mit entsprechend aktivierten oder gegebenenfalls N-geschützten enantiomerenreinen Aminosäuren in die entsprechenden Ester oder Amide, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäure- oder Alkoholrestes zur Trennung der Isomeren genutzt werden, indem man eine Trennung der nunmehr vorliegenden Diastereomeren durch Kristallisation oder Chromatographie an geeigneten Stationärphasen vornimmt und danach den mitgeführte chiralen Molekülteil mittels geeigneter Methoden wieder abspaltet.

Saure oder basische Produkte der Verbindung der Formel I können in Form ihrer Salze oder in freier Form vorliegen. Bevorzugt sind pharmakologisch verträgliche Salze, beispielsweise Alkali- oder Erdalkalimetallsalze bzw. Hydrochloride, Hydrobromide, Sulfate, Hemisulfate, alle möglichen Phosphate sowie Salze der Aminosäuren, natürlicher Basen oder Carbonsäuren.

Die Herstellung physiologisch verträglicher Salze aus zur Salzbildung befähigten Verbindungen der Formel I, einschließlich deren stereoisomeren Formen, gemäß Verfahrensschritt d) erfolgt in an sich bekannter Weise. Die Verbindungen der Formel I bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin, Diethanolamin oder Triethanolamin, Trometamol oder auch basischen Aminosäuren, etwa Lysin, Ornithin oder Arginin, stabile

Alkali-, Erdalkali- oder gegebenenfalls substituierte Ammoniumsalze. Sofern die Verbindungen der Formel I basische Gruppen aufweisen, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Phosphor-, Methansulfon-, Benzolsulfon-, p-Toluolsulfon-, 4-Brombenzol-sulfon-, Cyclohexylamidosulfon-, Trifluormethylsulfon-, Essig-, Oxal-, Wein-, Bernstein-, Glycerolphosphor-, Milch-, Äpfel-, Adipin-, Citronen-, Fumar-, Malein-, Glucon-, Glucuron- oder Trifluoressigsäure in Frage.

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel I und/oder eine gegebenenfalls stereoisomere Form der Verbindung der Formel I, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen zur selektiven Prophylaxe und Therapie all solcher Erkrankungen, an deren Verlauf eine verstärkte Aktivität der Metalloproteinasen beteiligt sind. Dazu gehören degenerative Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen. Ferner gehören dazu auch Erkrankungen des Bindegewebes wie Kollagenosen, Periodontalerkrankungen, Wundheilungsstörungen und chronische Erkrankungen des Bewegungsapparates wie entzündliche, immunologisch oder stoffwechselbedingte akute und chronische Arthritiden, Arthropathien, Myalgien und Störungen des Knochenstoffwechsels. Ferner eignen sich die Verbindungen der Formel I zur Behandlung der Ulceration, Atherosklerose und Stenosen. Weiterhin eignen sich die Verbindungen der Formel I zur Behandlung von Entzündungen, Krebserkrankungen, Tumormetastasenbildung, Kachexie, Anorexie, Herzversagen und septischem Schock. Ebenso eignen sich die Verbindungen zur Prophylaxe von Myocard- und Cerebral-Infarkten.

Die Applikation der erfindungsgemäßen Arzneimittel kann durch orale, inhalative, rektale oder transdermale Applikation oder durch subkutane, intraartikuläre, intraperitoneale oder intravenöse Injektion erfolgen. Bevorzugt ist die orale Applikation.

Die Erfindung betrifft auch ein Verfahren zur Herstellung eines Arzneimittels, das **dadurch gekennzeichnet, dass** man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuss- oder Sesamöl, Polyethylen-Glykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.

Für die Behandlung eines erwachsenen, etwa 70 kg schweren Patienten sind je nach Wirksamkeit der Verbindung gemäß Formel I, Tagesdosen von etwa 20 mg bis 1000 mg Wirkstoff, bevorzugt etwa 100 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Endprodukte werden in der Regel durch massenspektroskopische Methoden (FAB-, ESI-MS) und $^{1}$H-NMR (400 MHz, in DMSO-D6)bestimmt, angegeben sind jeweils der Hauptpeak oder die beiden Hauptpeaks. Temperaturangaben in Grad Celsius, RT bedeutet Raumtemperatur (22 °C bis 26 °C). Verwendete Abkürzungen sind entweder erläutert oder entsprechen den üblichen Konventionen.

Nachfolgend ist die Erfindung an Hand von Beispielen näher erläutert.

1-Arylsulfonyl-octahydro-cyclopenta[b]pyrrol-2-carbonsäure

Allgemeine-Vorschrift 1:

Carbonsäure (6,45 mmol) wird in 20 ml Dimethylformamid (DMF) gelöst und bei 0 °C mit 3 Äquivalenten einer 3N NaOH-Lösung (6,45 ml) versetzt. Nach 10 min tropfte man eine Lösung des Arylsulfonylchlorids (1,1 Äquivalente,

7,1 mmol) in 10 bis 15 ml DMF langsam zu, nach dem Erreichen der Raumtemperatur wurde der Ansatz noch für maximal 12 Stunden (h) bei Temperaturen zwischen 20 ˚C und 80 ˚C gerührt und das Lösungsmittel unter verminderten Druck entfernt. Das Rohprodukt wurde chromatographisch gereinigt

Beispiele:

Verbindung Nr. 4:1-[4-(1,1-Dimethyl-propyl)-benzolsulfonyl]-octahydro-cyclopenta[b]pyrrole-2-carbonsäure

1g Octahydro-cyclopenta[b]pyrrole-2-carbonsäure (6,45 mmol) wurde in 20 ml DMF gelöst und bei 0 ˚C mit 3 Äquivalenten einer 3N NaOH-Lösung (6,45 ml) versetzt. Nach 10 Minuten (min) tropfte man eine Lösung von 1,75 g 4-(1,1-Dimethyl-propyl-benzolsulfonylchlorid (1,1 Äquivalente, 7,1 mmol) in 12 ml DMF langsam zu, nach dem Erreichen der Raumtemperatur (RT) wurde der Ansatz noch für 6 h bei 40 ˚C gerührt und danach wurde das Lösungsmittel unter verminderten Druck entfernt. Das Rohprodukt wurde chromatographisch gereinigt.

Verbindung Nr. 20:1-(4-Acetyl-benzolsulfonyl)-octahydro-cyclopenta[b]pyrrole-2-carbonsäure

1 g Octahydro-cyclopenta[b]pyrrole-2-carbonsäure (6,45 mmol) wurde in 20 ml DMF gelöst und bei 0 ˚C mit 3 Äquivalenten einer 3 N NaOH-Lösung (6,45 ml) versetzt. Nach 10 min tropfte man eine Lösung von 1,55 g 4-Acetyl-benzolsulfonylchlorid (1,1 Äquivalente, 7,1 mmol) in 8 ml DMF langsam zu, nach dem Erreichen der RT wurde der Ansatz noch für 6 h bei 40 ˚C gerührt und danach wurde das Lösungsmittel unter verminderten Druck entfernt. Das Rohprodukt wurde chromatographisch gereinigt.

1-Arylsulfonyl-octahydro-cyclopenta[b]pyrrole-2-carbonsäure-hydroxyamid

Allgemeine Vorschrift 2:

Die sulfonierte Carbonsäure wurde in 10 ml DMF gelöst und mit 1,1 Äquivalenten Ethylchloroformiat, 2,2 Äquivalenten N-Ethylmorpholin sowie 3 Äquivalenten Trimethylsilylhydroxylamin versetzt. Nachdem man für mindestens 4 h auf 80 ˚C erhitzt hatte, entfernte man das Lösungsmittel unter verminderten Druck und reinigte das Rohprodukt mit chromatographischen Methoden.

Beispiele

Verbindung Nr. 50: 1-(Naphthalin-1-sulfonyl)-octahydro-cyclopenta[b]pyrrole-2-carbonsäure-hydroxyamide

200 mg (0,56 mmol) 1-(Naphthalin-1-sulfonyl)-octahydro-cydopenta[b]pyrrole-2-carbonsäure wurden in 10 ml DMF gelöst und mit 0,61 mmol Ethylchloroformiat, 1,23 mmol N-Ethylmorpholin sowie 1,68 mmol Trimethylsilylhydroxylamin versetzt. Nachdem man für 6 h auf 80 ˚C erhitzt hatte, entfernte man das Lösungsmittel unter verminderten Druck und reinigte das Rohprodukt mit chromatographischen Methoden.

Verbindung Nr. 52: 1-(4-Methanesulfonyl-benzolsulfonyl)-octahydro-cyclopenta[b]pyrrole-2-carbonsäure-hydroxyamid

200 mg (0,55 mmol) 1-(4-Methanesulfonyl-benzolsulfonyl)-octahydro-cyclopenta[b]-pyrrol-2-carbonsäure wurden in 10 ml DMF gelöst und mit 0,60 mmol Ethylchloroformiat, 1,22 mmol N-Ethylmorpholin sowie 1,67 mmol Trimethylsilylhydroxylamin versetzt. Nachdem man für 4 h auf 80˚C erhitzt hatte, entfernte man das Lösungsmittel unter verminderten Druck und reinigte das Rohprodukt mit chromatographischen Methoden.

Spezielle Vorschriften:

Verbindung Nr. 27: 1-(4'-Amino-biphenyl-4-sulfonyl)-octahydro-cyclopenta[b]pyrrole-2-carbonsäure

1 g 1-(4'-Nitro-biphenyl-4-sulfonyl)-octahydro-cyclopenta[b]pyrrol-2-carbonsäure (27) wurde in 15 ml DMF gelöst, mit 0,1 g Hydrierkatalysator (10% Pd auf Aktivkohle) versetzt und innerhalb von 2 h quantitativ hydriert. Nach der Entfernung des Lösungsmittels wurde das Rohprodukt chromatographisch gereinigt.

Verbindung Nr. 37:1-(4-Aminobenzolsulfonyl)-octahydro-cyclopenta[b]pyrrole-2-carbonsäure

1 g 1-(4-Nitrobenzol-sulfonyl)-octahydro-cyclopenta[b]pyrrol-2-carbonsäure (14) wurde in 15 ml DMF gelöst,

mit 0,1 g Hydrierkatalysator (10% Pd auf Aktivkohle) versetzt und innerhalb von 2 h quantitativ hydriert. Nach der Entfernung des Lösungsmittels wurde das Rohprodukt chromatographisch gereinigt.

Verbindung Nr. 22: 1-(4'-Isopropoxycarbonylamino-biphenyl-4-sulfonyl)-octahydro-cyclopenta[b]pyrrol-2- carbonsäure

Man löste 500 mg (1,30 mmol) 1-(4'-Amino-biphenyl-4-sulfonyl)-octahydro-cyclopenta[b]-pyrrol-2- carbonsäure in 3 ml DMF, kühlte im Eisbad auf 0 ˚C ab und fügte 2,6 mmol Pyridin hinzu. Nach 15 min Rühren bei 0 ˚C erfolgte die Zugabe von 2 mmol Isopropylchloroformiat in 3 ml DMF. Die Reaktionslösung rührte man anschließend noch für 2 h bei RT. Die Aufreinigung des Rohproduktes erfolgte mit Hilfe chromatographischer Methoden.

Verbindung Nr. 32: 1-(4'-Cyclopropylmethoxycarbonylamino-biphenyl-4-sulfonyl)-octahydro-cyclopenta[b]pyrrol-2-carbonsäure

Man löste 500 mg (1,30 mmol) 1-(4'-Amino-biphenyl-4-sulfonyl)-octahydro-cyclopenta[b]-pyrrol-2- carbonsäure in 3 ml DMF, kühlte im Eisbad auf 0 ˚C ab und fügte 2,6 mmol Pyridin hinzu. Nach 15. min Rühren bei 0 ˚C erfolgte die Zugabe von 2 mmol Cyclopropylmethylchloroformiat in 3 ml DMF. Die Reaktionslösung rührte man anschließend noch für 6 h bei RT. Die Aufreinigung des Rohproduktes erfolgte mit Hilfe chromatographischer Methoden.

Verbindung Nr. 23: 1-(4'-Methansulfonylamino-biphenyl-4-sulfonyl)-octahydro-cyclopenta[b]pyrrol-2- carbonsäure

Man löste 500 mg (1,30 mmol) 1-(4'-Amino-biphenyl-4-sulfonyl)-octahydro-cyclopenta[b]-pyrrol-2- carbonsäure in 3 ml DMF, kühlte im Eisbad auf 0˚C ab und fügte 2,6 mmol Pyridin hinzu. Nach 15 min Rühren bei 0 ˚C erfolgte die Zugabe von 1,40 mmol Methahsulfonylchlorid in 3 ml DMF. Die Reaktionslösung rührte man anschließend noch für 6 h bei Raumtemperatur. Die Aufreinigung des Rohproduktes erfolgte mit Hilfe chromatographischer Methoden.

Verbindung Nr. 25:1-(4'-Benzolsulfonylamino-biphenyl-4-sulfonyl)-octahydro-cyclopenta[b]pyrrol-2-carbonsäure

Man löste 500 mg (1,30 mmol) 1-(4'-Amino-biphenyl-4-sulfonyl)-octahydro-cyclopenta[b]-pyrrol-2-carbonsäure in 3 ml DMF, kühlte im Eisbad auf 0 ˚C ab und fügte 2,6 mmol Pyridin hinzu. Nach 15 min Rühren bei 0 ˚C erfolgte die Zugabe von 1,5 mmol Benzolsulfonylchlorid in 6 ml DMF. Die Reaktionslösung rührte anschließend noch für 12 h bei RT. Die Aufreinigung des Rohproduktes erfolgte mit Hilfe chromatographischer Methoden.

Verbindung Nr. 24: 1-(4'-Benzoylamino-biphenyl-4-sulfonyl)-octahydro-cyclopenta[b]pyrrol-2-carbonsäure

Man löste 500 mg (1,30 mmol) 1-(4'-Amino-biphenyl-4-sulfonyl)-octahydro-cyclopenta[b]-pyrrol-2-carbonsäure in 3 ml DMF, kühlte im Eisbad auf 0 ˚C ab und fügte 2,6 mmol Pyridin hinzu. Nach 15 min Rühren bei 0 ˚C erfolgte die Zugabe von 1,8 mmol Benzoylchlorid in 3 ml DMF. Die Reaktionslösung rührte anschließend noch für 20 h bei RT. Die Aufreinigung des Rohproduktes erfolgte mit Hilfe chromatographischer Methoden.

Verbindung Nr. 26: 1-[4'-(3-Phenyl-ureido)-biphenyl-4-sulfonyl]-octahydro-cyclopenta[b]pyrrol-2-carbonsäure

Man löste 500 mg (1,30 mmol) 1-(4'-Amino-biphenyl-4-sulfonyl)-octahydro-cyclopenta[b]-pyrrol-2- carbonsäure in 3 ml DMF, kühlte im Eisbad auf 0 ˚C ab und fügte 1,4 mmol Phenylisocyanat hinzu. Die Reaktionslösung rührte anschließend noch für 4 h bei RT. Die Aufreinigung des Rohproduktes erfolgte mit Hilfe chromatographischer Methoden.

Herstellungsbeispiele

| Nr. | Struktur | MS (ESI+) | NMR |
|---|---|---|---|
| 1 | 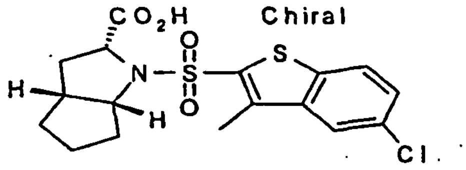 | 399,92<br>400,10 | 1,4-1,85: m, 6H; 1,9: m, 1H; 2,35: m, 1H; 2,55: m, 1H; 2,7: s, 3H; 4,0: m, 1H; 4,2: m, 1H; 7,6: d, 1H; (,1: m, 2H; 12.1, bs, 1H |
| 2 | 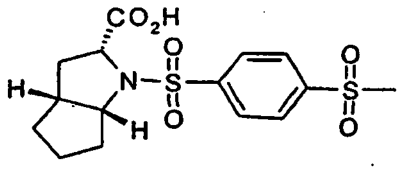 | 373,45<br>374,08 | 1,3-1,9: m, 8H; 2,15: m, 1H; 3,95: m, 1H; 3,3: s, 3H; 4,2: m, 1H; 8,1: d, 2H; 8,2: d, 2H; 12,5: bs, 1H |
| 3 | 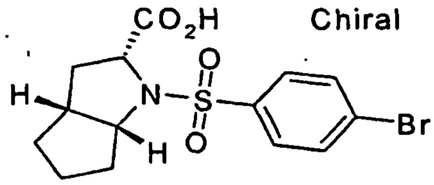 | 374,26<br>374,06 | 1,4-1,9: m, 7H; 2,15: m, 1H; 2,45: m, 1H; 3,9: m, 1H; 4,1: m, 1H; 7,75: d, 2H; 7,85: d, 2H 13,0: bs, 1H |
| 4 | 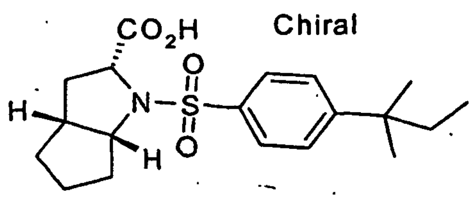 | 365,9<br>366,19 | 0,6: t, 3H; 1,3: s, 6H; 1,35-1,8: m, 8H; 1,9: m, 1H; 2,1: m, 1H; 2,4: m, 1H; 3,85: m, 1H; 4,0: m, 1H; 7,55: d, 2H; 7,75: d, 2H; 12,8: bs, 1H |
| 5 | 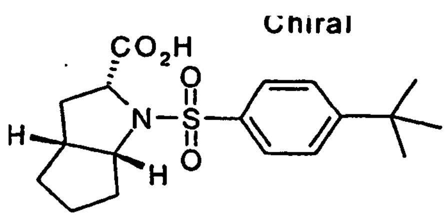 | 351,47<br>352,15 | 1,45: s, 9H; 1,5-1,8: m, 6H; 1,9: m, 1H; 2,15: m, 1H; 2,45: m, 1H; 3,85: m, 1H; 4,05: m, 1H; 7,6: d, 2H; 7,85: d, 2H; 12,5: s, 1H |
| 6 | 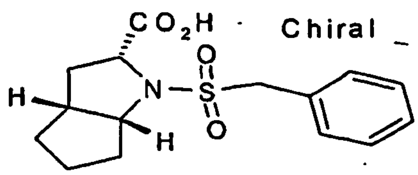 | 309,39<br>310,22 | 1,35: m, 2H; 1,7: m, 5H; 2,3: m, 1H; 2,65: m 1H; 4,15: m, 2H; 4,5: m, 2H; 7,4: m, 5H |
| 7 | 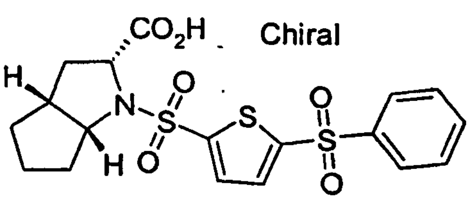 | 441,55<br>442,11 | 1,3-1,9: m, 7H; 2,2: m, 1H; 2,55: m, 1H; 4,0: m, 1H; 4,2: m, 1H; 7,6: m, 2H; 7,8: m, 2H; 7,95: m, 1H; 8,05: m, 2H; 13.0: bs, 1H |

(fortgesetzt)

| Nr. | Struktur | MS (ESI+) | NMR |
|---|---|---|---|
| 8 | 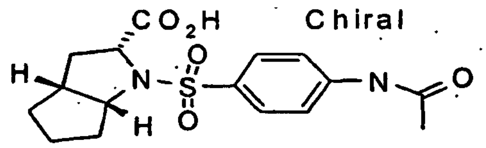 Chiral | 352,01<br>353,14 | 1,3-1,8: m, 6H; 1,9: m, 1H; 2,1: s, 3H; 2,15: m, 1H; 2,45: m, 1H; 3,85: m, 1H; 4,0: m, 1H; 7,8: m, 4H; 10,9: s, 1H |
| 9 | 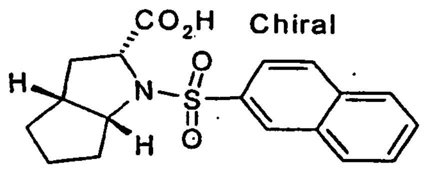 Chiral | 345,42<br>346,10 | 1,3-,18: m, 6H; 1,9:m, 1H; 2,1: m, 1H; 2,4: m, 1H; 3,95: m, 1H; 4,2: m, 1H; 7,7: m, 2H; 7,9: d, 1H; 8,05: d, 1H; 8,15: d, 1H; 8,2: d, 1H; 8,5: s, 1H; 12,8: bs, 1H |
| 10 | 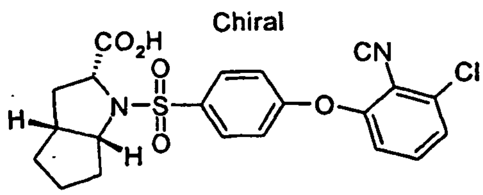 Chiral | 446,91<br>447,11 | 1,3-1,8: m, 6H; 1,9: m, 1H; 2,15: m, 1H; 2,45: m, 1H; 3,9: m, 1H; 4,1: m, 1H; 7,2: d, 1H; 7,4: d, 2H; 7,6: d, 1H; 7,75: m, 2H; 7,9: d, 2H |
| 11 | 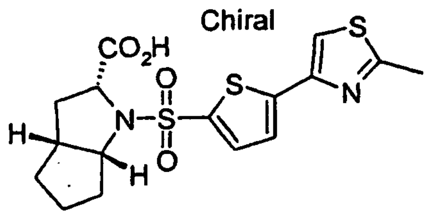 Chiral | 398,52<br>399,02 | 1,3-1,8: m, 6H; 1,95: m, 1H; 2,2: m, 1H; 2,5: m, 1H; 2,7: s, 3H; 3,95: m, 1H; 4,05: m, 1H; 7,65: s, 2H; 8,1: s, 1H |
| 12 | 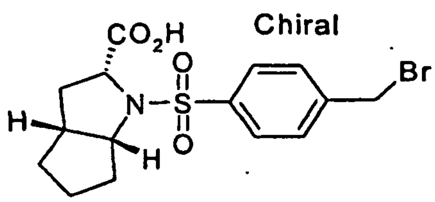 Chiral | 388,28<br>388,04 | 1,3-1,8: m, 6H; 1,9: m, 1H; 2,1: m, 1H; 2,45: m, 1H; 3,9 m, 1H; 4,1: m, 1H; 4,4: s, 2H; 7,7: d, 2H; 7,85: d, 2H |
| 13 | 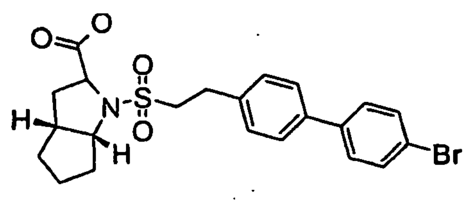 | 478,41<br>480,04 | 1,3-1,9: m, 7H; 2,4: m, 1H; 2,7: m, 1H; 3,05: t, 2H; 3,45: m, 2H; 4,25: m, 1H; 4,35: m, 1H; 7,35: m, 2H; 7,65: m, 6H |
| 14 | 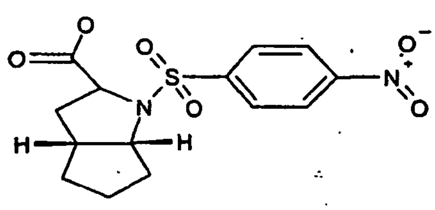 | 340,36<br>341,04 | 1,3-1,9: m, 7H; 2,1: m, 1H; 2,45: m, 1H; 3,9: m, 1H; 4,2: m, 1H; 8,1: d, 2H; 8,4: d, 2H |

(fortgesetzt)

| Nr. | Struktur | MS (ESI+) | NMR |
|---|---|---|---|
| 15 | 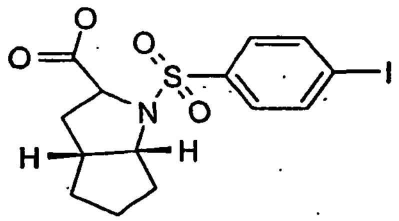 | 421,26<br>421,97 | 1,3-1,8: m, 6H; 1,9: m, 1H; 2,1: m, 1H; 2,45: m, 1H; 3,8: m, 1H; 4,05: m, 1H; 7,55: d, 1H; 8,05: d, 1H |
| 16 | 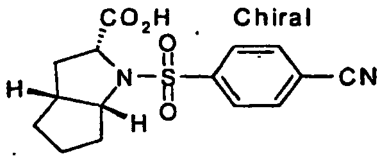 | 320,37<br>321,10 | 1,3-1,9: m, 7H; 2,2: m, 1H; 2,45: m, 1H; 3,95: m, 1H; 4,2: m, 1H; 8,0: d, 2H; 8,1: d, 2H; 12,8: s, 1H |
| 17 | 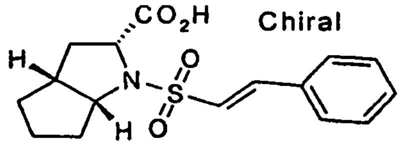 | 321,39<br>322,02 | 1,35-1,8: m, 6H; 1,95: m, 1H; 2,4: m, 1H; 2,65: m, 1H; 4,05: m, 1H; 4,2: m, 1H; 7,4: s, 2H; 7,45: m, 3H; 7,7: m, 2H |
| 18 | 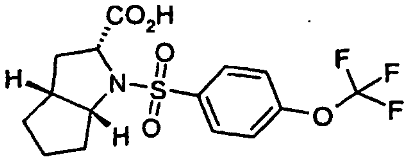 | 379,36<br>379,97 | 1,3-1,9: m, 7H; 2,15: m, 1H; 2,5: m, 1H; 3,9: m, 1H; 4,1: m, 1H; 7,6: d, 2H; 8,0: d, 2H; 12,8: s, 1H |
| 19 | 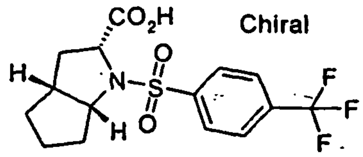 | 363,36<br>364,00 | 1,3-1,9: m, 7H; 2,2: m, 1H; 2,5: m, 1H; 4,0: m, 1H; 4,2: m, 1H; 8,1: d, 2H; 8,15: d, 2H; 12,95: s, 1H |
| 20 | 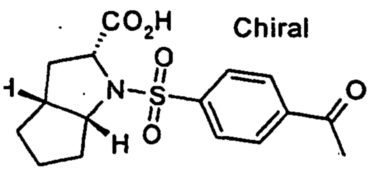 | 337,39<br>338,05 | 1,3-1,8: m, 6H; 1,9: m, 1H; 2,15: m, 1H; 2,45: m, H; 2,65: s, 3H; 3,9: m, 1H; 4,15: m, 1H; 7,95: d, 2H; 8,15: d, 2H; 12,8: s, 1H |
| 21 | 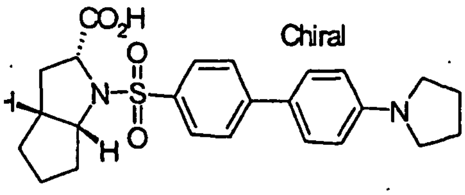 | 440,56<br>441,09 | 1,3-1,8: m, 6H; 1,9: m, 1H; 2,0: m, 4H; 2,15: m, 1H; 2,45: m, 1H; 3,3: m, 4H; 3,9: m, 1H; 4,05: m, 1H; 6,6: d, 2H; 7,6: d, 2H; 7,8: m, 4H |

(fortgesetzt)

| Nr. | Struktur | MS (ESI+) | NMR |
|---|---|---|---|
| 22 | 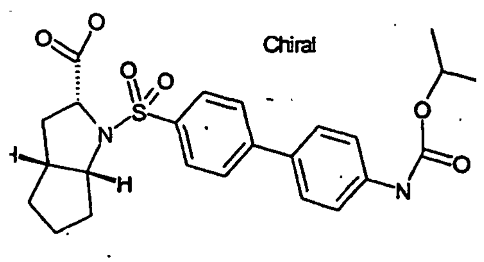 | 472,56<br>473,16 | 11,3-1,8: m, 6H; 1,35: d, 6H; 1,9: m, 1H; 2,2: m, 1H; 2,45: m, 1H; 3,9: s, 1H; 4,0: s, 1H; 4,9: s, 1H; 7,6: d, 2H; 7,8: d, 2H; 7,8: m, 4H; 10,0: s, 1H |
| 23 | 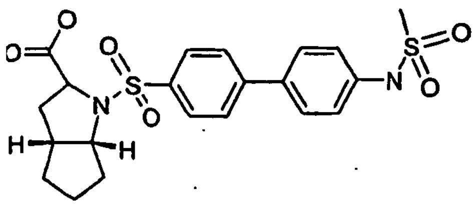 | 464,56<br>465,20 | 1,3-1,9: m, 7H; 2,1: m, 1H; 2,4: m, 1H; 3,0: s, 3H; 3,9: s, 1H; 4,05: s, 1H; 7,35: d, 2H; 7,8: d, 2H; 7,9: m, 4H; 9,9: s, 1H |
| 24 | 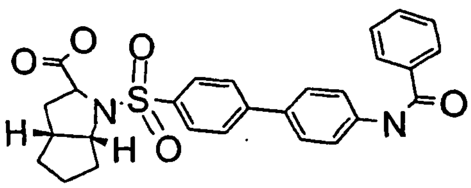 | 490,58<br>491,34 | 1,3-1,9: m, 7H; 2,1: m, 1H; 2,4: m, 1H; 3,95: m, 1H; 4,1: m, 1H; 7,55: m, 2H; 7,8-8,1: m, 8H; 8,4: m, 2H |
| 25 | 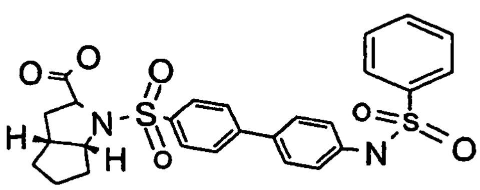 | 526,63<br>527,12 | 1,3-1,8: m, 6H; 1,8: m, 1H; 2,0: m, 1H; 2,1: m, 1H; 3,85: m, 1H; 4,05: m, 1H; 7,2: m, 1H; 7,6: m, 6H; 7,9: m, 6H |
| 26 | 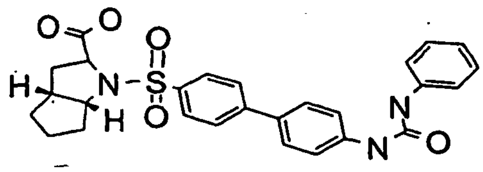 | 505,59<br>506,28 | 1,3-1,8: m, 6H; 1,9: m, 1H; 2,15: m, 1H; 2,45: m, 1H; 3,9: m, 1H; 4,1: m, 1H; 7,0-8,5: m, 13H; 12,8: s, 1H |
| 27 | 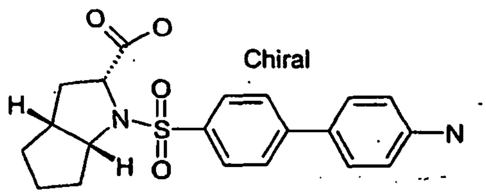 | 386,47<br>387,14 | 1,3-1,8: m, 6H; 1,85: m, 1H; 2,1: m, 1H; 2, 4: m, 1H; 3,85: m, 1H; 4,1: m, 1H; 8,1: d, 2H; 8,3: d, 2H |

(fortgesetzt)

| Nr. | Struktur | MS (ESI+) | NMR |
|---|---|---|---|
| 28 | 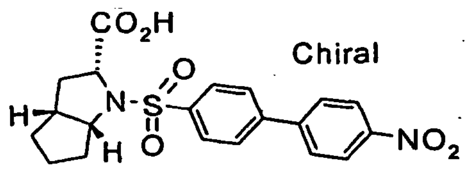 | 416,46<br>417,23 | 1,3-1,8: m, 6H; 1,9: m, 1H; 2,15: m 1H; 2,45: m, 1H; 3,95: m, 1H; 4,10: m, 1H; 8,0: m, 4H; 8,1: d, 2H; 8,3: d, 2H |
| 29 | 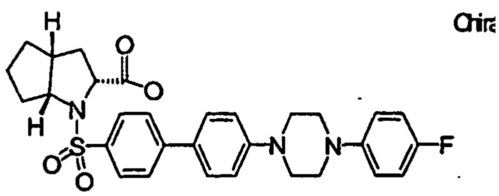 | 549,67<br>550,37 | 1,3-1,8: m, 6H; 1,9: m, 1H; 2,15: m, 1H; 2,45: m, 1H; 3,25: m, 4H; 3,4: m, 4H; 3,9: m, 1H; 4,05: m, 1H; 7,1: m, 6H; 7,7: d, 2H; 7,9: m, 4H; |
| 30 | 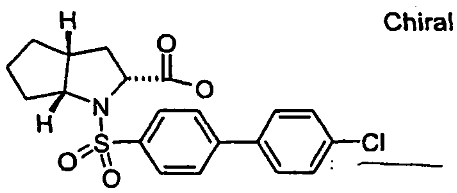 | 405,90<br>406,21 | 1,3-1,8: m, 6H; 1,9: m, 1H; 2,2: m, 1H; 2,45: m, 1H; 3,9: m, 1H; 4,1: m, 1H; 7,6: d, 2H; 7,8: d, 2H; 7,95: m, 4H |
| 31 | 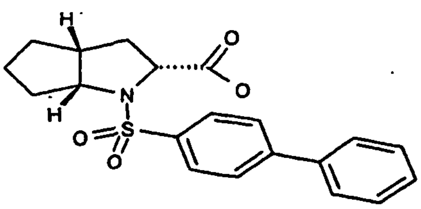 | 371,46<br>372,16 | 1,3-1,8: m, 6H; 1,9: m, 1H; 2,2: m, 1H; 2,45: m, 1H; 3,9: m, 1H; 4,1: m, 1H; 7,5: m, 3H; 7,75: m 2H; 7,95: m, 4H |
| 32 | 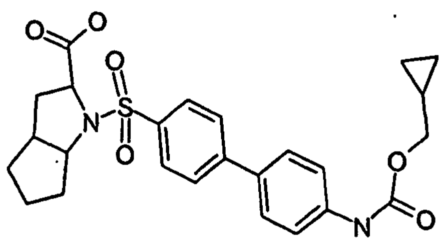 | 484,58<br>485,12 | 0,35: m, 2H; 0,55: m, 2H; 1,3-1,8: m, 8H; 1,95: m, 1H; 2,3: m, 1H; 3,95: 1H; 4,2: m, 1H; 7,6: m, 3H; 7,85: m, 3H;. 8,05: m, 2H |
| 33 | 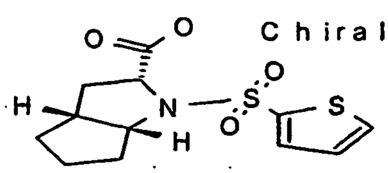 | 301,39<br>302,06 | 1,3-1,8: m, 6H; 1,9: m, 1H; 2,2: m, 1H; 2,4: m, 1H; 3,9: m, 1H; 4,05: m, 1H; 7,3: m, 1H; 7,7: m, 1H; 8,0: m, 1H |
| 34 | 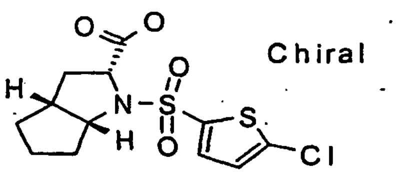 | 335,83<br>336,01 | 1,3-1,8: m, 6H; 1,9: m, 1H; 2,15: m, 1H; 2,5: m, 1H; 3,95: m ,1H; 4,: m, 1H; 7,35: m, 1H; 7,35: d, 1H; 7,65: d, 1H |

(fortgesetzt)

| Nr. | Struktur | MS (ESI+) | NMR |
|---|---|---|---|
| 35 | 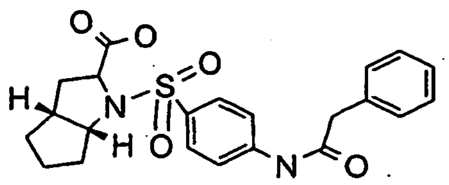 | 428,51<br>429,24 | 1,3-1,7: m, 6H; 1,9: m, 1H; 2,1: m, 1H; 2,4: m, 1H; 3,8: m, 1H; 4,0: m, 1H; 7,3: m, 5H; 7,8: d, 2H; 7,85: d, 2H,10,8: s, 1H |
| 36 | 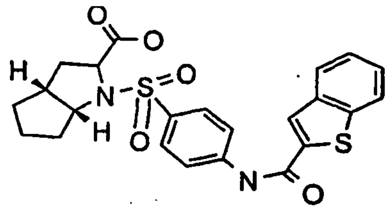 | 470,57<br>471,22 | 1,3-1,7: m, 6H; 1,85: m, 1H; 2,1: m, 1H; 2,45: m, 1H; 3,9: m, 1H; 4,1: m, 1H; 7,5: m, 2H; 7,9: d, 2H; 8,05: m, 4H; 8,45: s, 1H; 10,9: s, 1H |
| 37 | 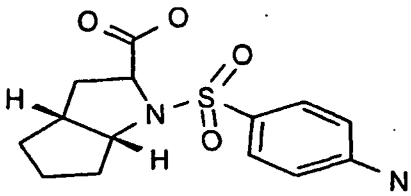 | 310,09<br>311,13 | 1,3-1,7: m, 6H; 1,85: m, 1H; 2,1: m, 1H; 2,4: m, 1H; 3,8: m, 1H; 3,9: m, 1H; 6,6: d, 2H; 7,45: d, 2H; |
| 38 | 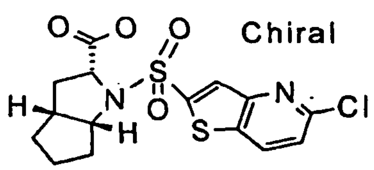 | 386,88<br>387,14 | 1,3-2,0: m, 7H; 2,2: m, 1H; 2,55: m, 1H; 4,2: m, 1H; 4,25: m, 1H; 7,65: d, 1H; 8,2: s, 1H; 8,7: d, 2H |
| 39 | 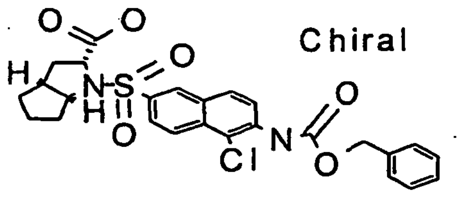 | 529,02<br>529,25 | 1,3-1,8: m, 6H; 1,8: m, 1H; 2,1: m, 1H; 2,4: m, 1H; 3,95: m, 1H; 4,2: m, 1H; 5,2: s, 2H; 7,4: m, 5H; 8,0: m, 2H; 8,2: d, 1H; 8,4: d, 1H; 8,55: s, 1H |
| 40 | 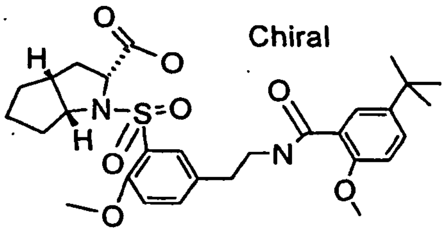 | 558,69<br>559,32 | 1,2: s, 9H; 1,3-1,7: m, 6H; 1,9: m, 1H; 2,25: m, 1H; 2,45: m, 1H; 2,85: m, 2H; 3,5: m, 2H; 3,65: m, 1H; 3,8: s, 3H; 3,9: s, 3H; 4,5: m, 1H; 7,0: d, 1H; 7,2: d, 1H; 7,45: m, 2H; 7,7: m, 2H; 8,15: m, 1H |

(fortgesetzt)

| Nr. | Struktur | MS (ESI+) | NMR |
|---|---|---|---|
| 41 | 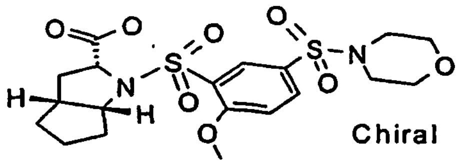 | 474,56<br>475,21 | 1,3-1,8: m, 6H; 2,0: m, 1H; 2,3: m, 1H; 2,6: m, 1H; 2,9: m, 4H; 3,6: m, 4H; 3,8: m, 1H; 4,1: s, 3H; 4,5: m, 1H; 7,5: d, 1H; 7,95: d, 1H; 8,05: d, 1H |
| 42 | 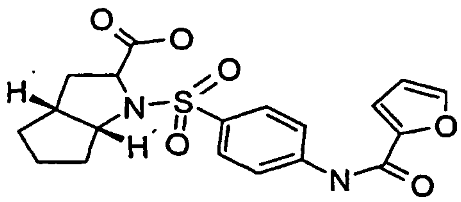 | 404,45<br>405,22 | 1,3-1,8: m, 6H; 1,85: m, 1H; 2,1:m, 1H; 2,45: m, 1H; 3,9: m, 1H; 4,05: m, 1H; 6,7: d, 1H; 7,4: d, 1H; 7,8: d, 2H; 8,0: m, 3H; 10,4: s, 1H |
| 43 | 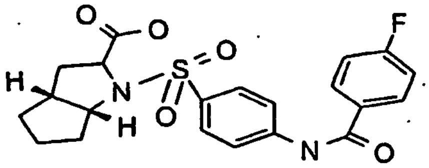 | 432,47<br>433,23 | 1,3-1,7: m, 6H; 1,85: m, 1H; 2,1: m, 1H; 2,45: m, 1H; 3,9: m, 1H; 4,05: m, 1H; 7,4: m, 2H; 7,8: m, 2H; 8,05: m, 4H; 10,6: s, 1H |
| 44 | 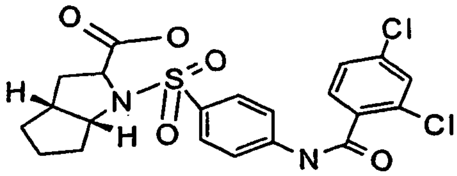 | 483,37<br>483,19 | 1,3-1,8: m, 6H; 1,85: m, 1H; 2,15: m, 1H; 2,45: m, 1H; 3,9: m, 1H; 4,05: m, 1H; 7,5: d, 1H; 7,65: d, 1H; 7,75: s, 1H; 7,8: d, 2H; 7,9: d, 2H; 10,9: s, 1H |
| 45 | 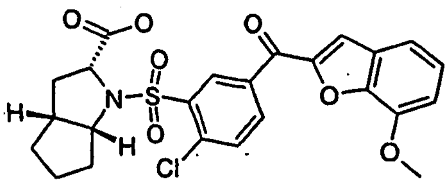 | 503,96<br>504,25 | 1,3-1,85: m, 6H; 2,05: m, 1H; 2,4: m, 1H; 2,65: m, 1H; 3,95: s, 3H; 4,0: m, 1H; 4,6: m, 1H; 7,2: d, 2H; 7,3: m, 1H; 7,4: d, 1H; 7,85: s, 1H; 7,9: d, 1H; 8,2: d, 1H; 8,45: s, 1H |
| 46 | 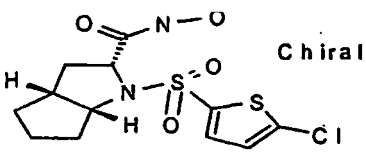 | 350,85<br>351,14 | 1,4-2.0: m, 8H; 2,4: m, 1H; 3,8: m, 1H 3,95: m, 1H; 7,35: d, 1H; 7,65: d, 1H 10,4: s, 1H |
| 47 | 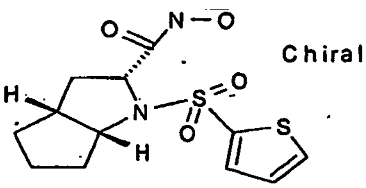 | 316,40<br>317,17 | 1,8-2,4: m, 8H; 2,85: m, 1H; 3,8: m, 1H; 3,9: m, 1H; 7,25: m, 1H; 7.8: m, 1H; 8,05: m, 1H; 10,4: s, 1H |

(fortgesetzt)

| Nr. | Struktur | MS (ESI+) | NMR |
|---|---|---|---|
| 48 | 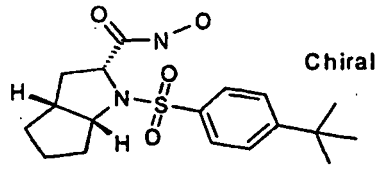 | 366,48<br>367,22 | 1,35: s, 9H; 1,4-1,95: m, 8H; 2,35: m, 1H; 3,8-4,0: m, 2H; 7.6: d, 2H; 7,8: d, 2H; 10,55: s, 1H |
| 49 | 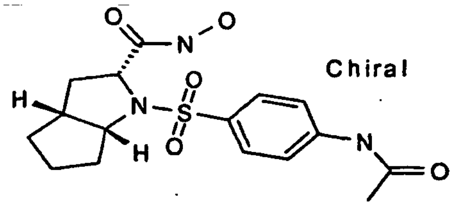 | 367,43<br>368,19 | 1,4-1,95: m, 8H; 2.1: s, 3H; 2,35: m, 1H; 3.8-4.0: m, 2H; 7.8: m, 4H, 10.4: s, 1H, 10.6: s, 1H |
| 50 | 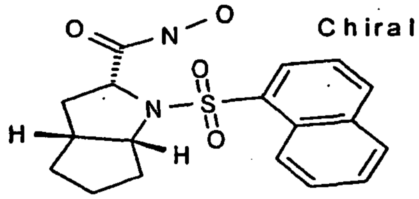 | 360,44<br>361,77 | 1,4-2,0: m, 8H; 2,35: m, 1H; 3,8-4,1: m, 2H; 7,65: m, 2H; 7,95: d, 1H; 8,10: d, 1H; 8,15: m, 2H; 8,5: s, 1H, 10.6: s, 1H |
| 51 | 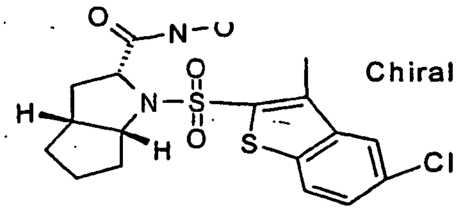 | 414,93<br>415,12 | 1,4-1,9: m, 7H; 1,95-2,2: m, 2H; 2,7: s, 3H; 4,1: m, 2H; 7,6: m, 1H; 8,1: m, 2H; 10,6: s,1H |
| 52 | 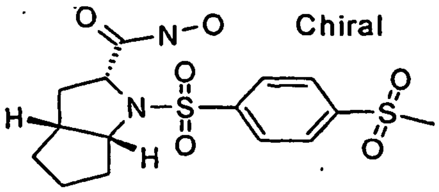 | 388,46<br>389,14 | 1,4-1,8: m, 8H; 1,90: m, 1H; 3,4: s, 3H; 3,9: m, 2H; 8,15: m, 4H; 10,6: s, 1H |
| 53 | 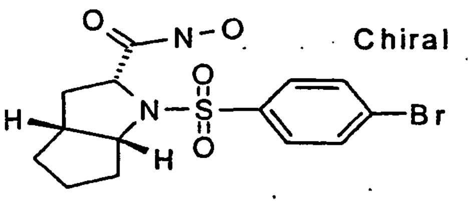 | 389,27<br>389,07 | 1,4-2,0: m, 8H; 2,40: m, 1H; 3,9: m, 2H; 7,8: m, 4H; 10,6: s, 1H |
| 54 | 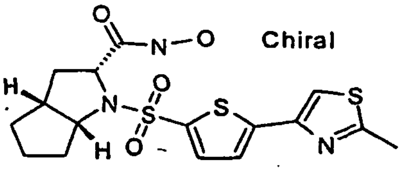 | 413,54<br>414,05 | N.A. |

(fortgesetzt)

| Nr. | Struktur | MS (ESI+) | NMR |
|---|---|---|---|
| 55 | 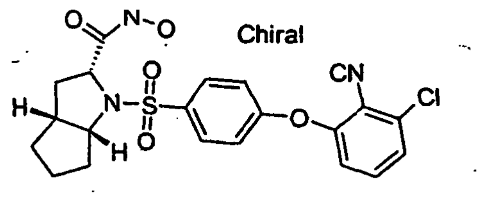 | 461,93<br>462,07 | 1,4-2,1: m, 8H; 2,4: m, 1H; 3,95: m, 2H; 7,65: d, 1H; 7,40: d; 1H; 7,50: d, 1H; 7,60: d, 1H; 7,75: m, 1H; 7,95: d, 2H; 10,6: s, 1H |
| 56 | 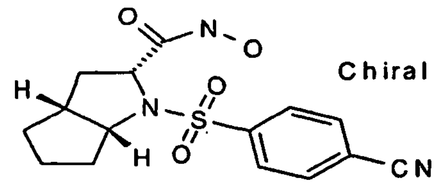 | 335,38<br>336,14 | 1,4-2,1: m, 8H; 2,4: m, 1H; 2,4: m, 1H; 3,95: m, 2H; 8,0: d, 2H; 8,1: d, 2H; 10,6: s, 1H |
| 57 | 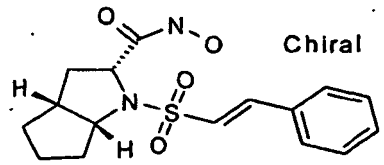 | 336,41<br>337,11 | 1,4-1,8: m, 6H; 2,0: m, 1H; 2,2: m, 1H; 2,6: m, 1H; 3,95: m, 1H; 4,05: m, 1H; 7,9: m, 4H; 7,7: m, 2H; 10,5: s, 1H |
| 58 | 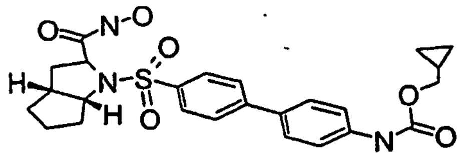 | 499,59<br>500,09 | 0,35; m, 2H; 0,55: m, 2H; 1,1-1,85: m, 9H; 1,95: m, 2H; 2,35: m, 1H; 3,85: m, 1H; 3,95: m, 1H; 7,60: d, 2H, 7,7: d, 2H; 7,9: m, 4H; 9,8: s, 1H; 10,6: s, 1H |
| 59 | 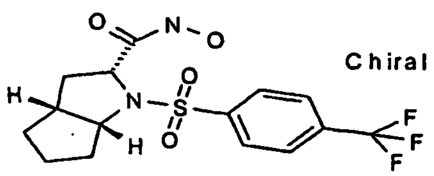 | 378,38<br>379,02 | 1,4-2,1: m, 8H; 2,4: m, 1H; 3,9: m, 2H; 8,0: d, 2H; 8,1: d, 2H; 10,65: s, 1H |
| 60 | 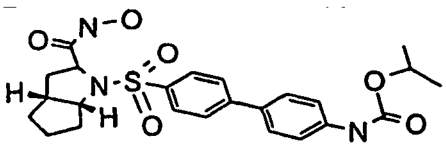 | 487,58<br>488,36 | 1,26: d, 6H; 1,4-2,0: m, 8H; 2,35: m, 1H; 3,85: m, 2H; 4,95: m, 1H; 7,6: d, 2H; 7,7: d, 2H; 7,85: m, 4H;9,8:s,1H;10,6:s,1H |
| 61 | 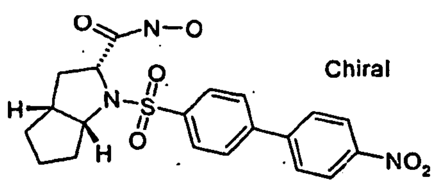 | 431,12<br>432,01 | 1,4-1,8: m, 8H; 2,45: m, 1H; 3,9: m, 2H; 8,05: m, 6H, 8,35: m, 2H; 10,65: s, 1H |

(fortgesetzt)

| Nr. | Struktur | MS (ESI+) | NMR |
|---|---|---|---|
| 62 | 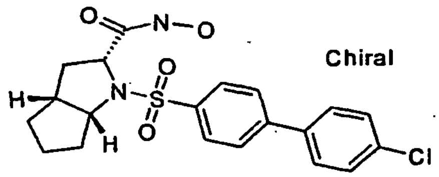 Chiral | 420,92<br>421,24 | 1,4-1,8: m, 6H; 2,0: m, 2H; 2,4: m, 1H; 3,95: m, 2H; 7,6: d, 2H; 7,8: d, 2H; 8,0: m, 4H; 10,6: s, 1H |
| 63 | 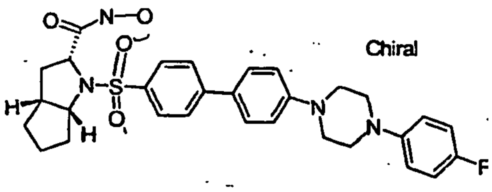 Chiral | 564,68<br>565,39 | 1,4-2,05: m, 8H; 2,35: m, 1H; 3,35: m, 4H; 3,40: m, 4H; 3,90: m, 2H; 7,1: m, 6H; 7,6: d, 2H; 7,9: m, 4H; 10,6: s, 1H |
| 64 | 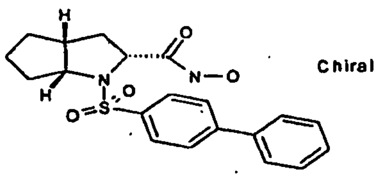 Chiral | 386,47<br>387,19 | 1,4-2,0: m, 8H; 2,45: m, 1H; 3,95: m, 2H; 7,45: m, 3H; 7,75: m, 2H; 7,95: m, 4H; 10,6: s, 1H |
| 65 | 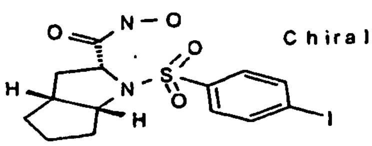 Chiral | 435,99<br>437,02 | 1,4-20: m, 8H; 2,35: m, 1H; 3,85: m, 2H; 7,6: d, 2H; 8,0: d, 2H; 10,6: s, 1H |
| 66 | 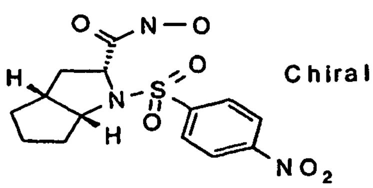 Chiral | 355,08<br>356,09 | 1,4-2,0: m, 8H; 2,35: m, 1H; 3,95: m, 2H; 8,15: d, 2H; 8,4: d, 2H; 10,6: s, 1H |
| 67 | 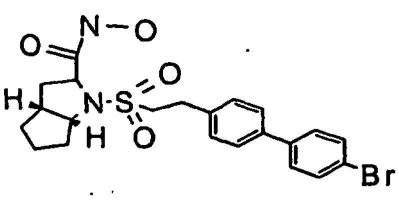 | 493,42<br>493,10 | 1,4-1,95: m, 8H; 2,35: m, 1H; 2,70: m, 2H; 3,0: m, 2H; 4,1: m, 1H; 4,3: m, 1H; 7,4: m, 2H; 7,6: m, 6H; 10,6: s, 1H |
| 68 | 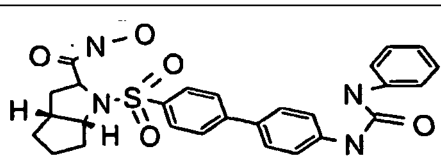 | 520,61<br>521,16 | 1,4-1,9: m, 8H; 2,4: m, 1H; 3,85: m, 2H; 7,0-8,0: m, 12H; 8,6: s, 1H; 8,8: s, 1H; 10,6: s, 1H |
| 69 | 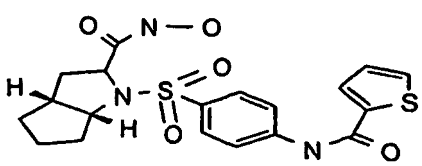 | 435,52<br>436,19 | 1,4-1,85: m, 6H; 1,9: m, 2H; 2,4: m, 1H; 3,95: m, 2H; 7,25: m, 1H; 7,8-8,1: m, 6H, 8,9: s, 1H; 10,5: s, 1H |

(fortgesetzt)

| Nr. | Struktur | MS (ESI+) | NMR |
|---|---|---|---|
| 70 | 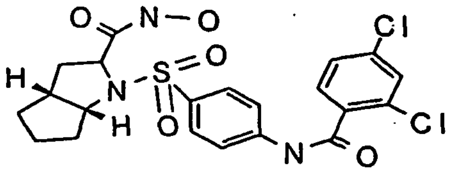 | 498,39<br>498,17 | 1,4-1,85: m, 6H; 1,9: m, 2H; 2,4: m, 1H; 3,85: m, 2H; 7,6-7,9: m, 7H; 8,95: s, 1H; 11,0: s, 1H |
| 71 | 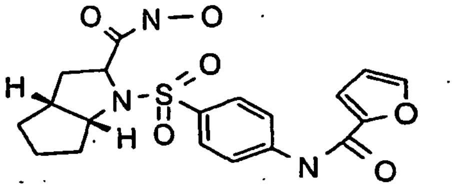 | 419,59<br>420,21 | 1,4-1,85: m, 6H; 1,95: m, 2H; 2,3: m, 1H; 3,95: m, 2H; 3,85: m, 2H; 6,7: m, 1H; 7,4: m, 1H; 7,8: m, 2H; 8,0: m, 3H; 8,9: s, 1H; 10,8: s, 1H |
| 72 | 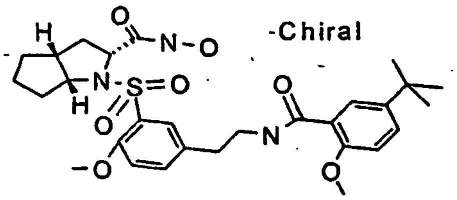 | 573,71<br>574,38 | 1,25: s, 9H; 1,3-1,8: m, 6H; 1,9: m, 1H; 2,1: m, 1H; 2,4: m, 1H; 2,85: m, 2H; 3,5: m, 2H; 3,8: s, 3H; 3,9: s, 3H; 4,3: m, 2H; 7,0: d, 2H; 7,2: d, 2H; 7,5: m, 2H; 7,7: d, 2H; 8,1: m, 1H; 10,2: s, 1H |
| 73 | 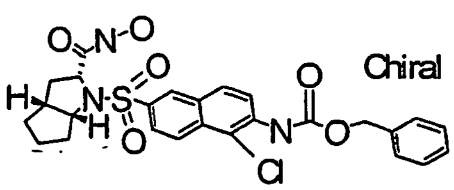 | 544,03<br>544,24 | 1,4-1,8: m, 6H; 1,95: m, 2H; 2,3: m, 1H; 3,95: m, 2H; 5,2: s, 2H; 7,4: m, 6H; 8,0: m, 2H; 8,2: d, 1H; 8,45: d, 1H; 8,55: s, 1H; 10,8: s, 1H |
| 74 | 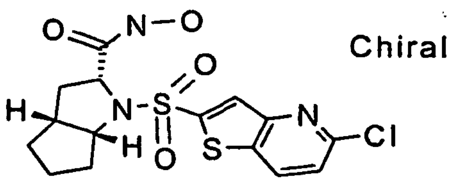 | 401,89<br>402,13 | 1,4-1,9: m, 6H; 2,05: m, 2H; 2,4: m, 1H; 3,95: m, 1H; 4,1: m, 1H; 7,6: d, 1H; 8,25: s, 1H; 8,7: d, 1H |
| 75 | 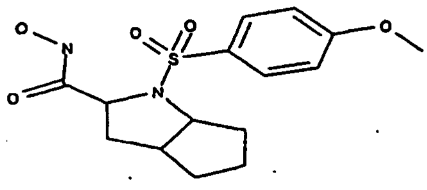 | 340,11 | 1,4-2,0: m,. 8H; 2,3: m, 1H; 3,8: m, 2 H; 3,85: s, 3H; 7,17: d, 2H; 7,82: d, 2 H; 8,9: s, 1H; 10,6: s, 1H. |
| 76 | 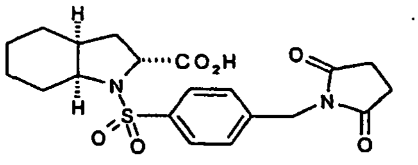 | 420,49<br>421,02 | N.A. |

(fortgesetzt)

| Nr. | Struktur | MS (ESI+) | NMR |
|---|---|---|---|
| 77 | 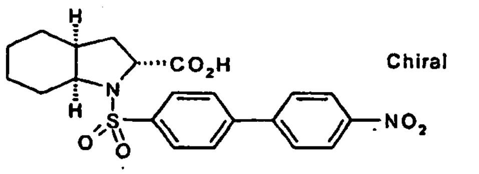 | 430,48<br>431,17 | 1,0-1,9: m, 9H; 1,85: m, 1H; 2,45: m, 1H; 3,75: m, 1H;<br>4,3: m, 1H; 8,0: m, 6H; 8,4: m, 2H |
| 78 | 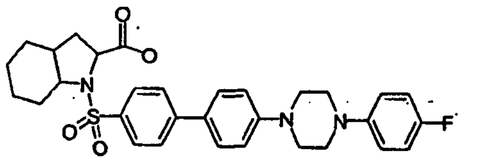 | 563,70<br>564,35 | 1,0-1,95: m, 10H; 2,4: m, 1H; 3,25: m, 4H; 3,45:m 4H; 3,75: m, 1H; 4,3: m, 1H; 7,1: m, 6H; 7,7: m, 2H; 7,85: m, 4H |
| 79 | 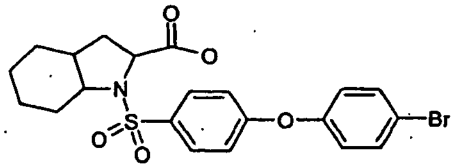 | 480,38<br>482,16 | 1,0-1,9: m, 9H; 2,35: m, 1H; 2,4: m, 1H; 3,7: m, 1H; 4,2: m, 1H; 7,1: m, 4H; 7,6: m, 2H; 7,8: m, 2H |
| 80 | 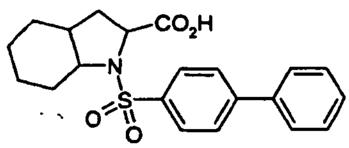 | 371,46<br>372,16 | 1,3-1,9: m, 9H; 2,2: m, 1H; 2,45: m, 1H; 3,9: m, 1H; 4,1: m, 1H; 7,5: m, 3H; 7,8: m, 3H; 7,95: m, 4H |
| 81 | 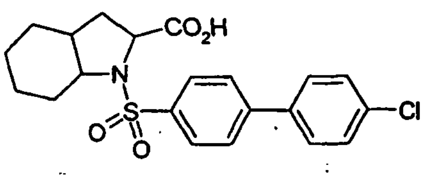 | 419,93<br>420,34 | 1,1-1,8: m, 9H; 1,9: m, 1H; 2,1: m, 1H; 3,65: m, 1H; 4,05: pt, 1H; 7,6: d, 2H; 7,8: d, 2H; 7,9: m, 4H |
| 82 | 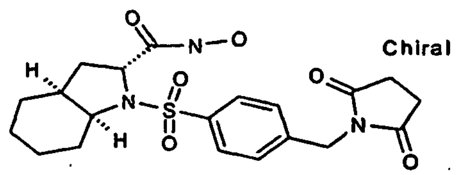 | 435,50<br>436,19 | 1,0-1,6: m, 8H; 1,9: m, 1H; 2,25: m,1H; 2,6: m, 1H; 2,75: m, 4H; 3,6: m, 1H; 4,1: m, 1H; 7,4: d, 2H; 7,8: d, 2H |
| 83 | 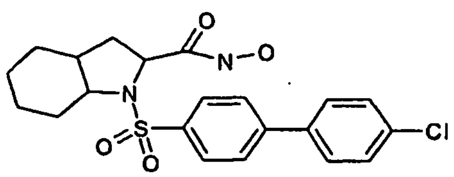 | 434,95<br>435,20 | 1,1-2,0: 11H; 3,6: m, 1H; 3,9: m, 1H; 7,6: d, 2H; 7,8: d, 2H; 7,95: d, 2H; 8,0: d, 2H |
| 84 | 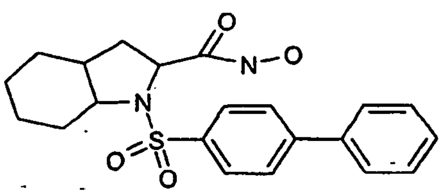 | 400,50<br>401,25 | 1,0-1,6: m, 8H; 1,9: m, 1H; 2,2: m, 1H; 2,7: m, 1H; 3,7: m, 1H; 4,15: m, 1H; 7,8: m, 4H; 8,0: m, 2H, 8,1: m, 2H |

Pharmakologische Beispiele

Darstellung und Bestimmung der enzymatischen Aktivität der katalytischen Domäne des humanen Stromelysins (MMP-3) und der Neutrophilen-Kollagenase (MMP-8). Die beiden Enzyme -Stromelysin (MMP-3) und Neutrophilen-Kollagenase (MMP-8) - wurden dargestellt nach Ye et al. (Biochemistry; 31 (1992) Seiten 11231-11235). Zur Messung der Enzymaktivität oder der Enzyminhibitorwirkung werden bei physiologischem pH 70 μl Pufferlösung, und 10 μl Enzymlösung mit 10 μl einer 10%igen (v/v) wässrigen Dimethylsulfoxid-Lösung, die gegebenenfalls den Enzyminhibitor enthält, für 15 Minuten inkubiert. Nach Zugabe von 10 μl einer 10%igen (v/v) wässrigen Dimethylsulfoxid-Lösung, die 1 mmol/l des Substrates enthält, wird die Enzymreaktion fluoreszenzspektroskopisch verfolgt (328 nm (ex) / 393 nm(em)).

Die Enzymaktivität wird dargestellt als Extinktionszunahme/Minute. Die in Tabelle 2 aufgeführten $IC_{50}$-Werte werden als diejenige Inhibitorkonzentrationen ermittelt, die jeweils zu einer 50%eigen Inhibierung des Enzyms führen. Die Pufferlösung enthält 0,05 % Brij (Sigma, Deisenhofen, Deutschland) sowie 0,1 mol/l Tris/HCl, 0,1 mol/l NaCl, 0,01 mol/l $CaCl_2$ und 0,1 mol/l Piperazin-N,N'-bis[2-ethan-sulfonsäure] (pH=7,5).
Die Enzymlösung enthält 5 μg/ml einer der nach Ye et al. dargestellten Enzymdomänen. Die Substratlösung enthält 1 mmol/l des fluorogenen Substrates (7-Methoxycoumarin-4-yl)acetyl-Pro-Leu-Gly-Leu-3-(2',4'-dinitrophenyl)-L-2,3-diaminopropionyl-Ala-Arg-$NH_2$ (Bachem, Heidelberg, Deutschland).

Bestimmung der enzymatischen Aktivität der katalytischen Domäne der humanen Kollagenase -3 (MMP-13).

Dieses Protein wird als inaktives Pro-Enzym von der Fa. INVITEK, Berlin, erhalten (Katalog Nr. 30 100 803).
Aktivierung des Proenzyms:
2 Volumenanteile Proenzym werden mit 1 Volumenanteil APMA-Lösung bei 37 ˚C für 1,5 Stunden inkubiert. Die APMA-Lösung wird aus einer 10 mmol/L p-Aminophenyl-Mercuric Acetate Lösung in 0,1 mmol/L NaOH durch Verdünnen mit 3 Volumenteile Tris/HCl Puffer pH7,5 (siehe unten) hergestellt. Der pH-Wert wird durch Zugabe von 1mmol/L HCl zwischen 7,0 und 7,5 eingestellt. Nach der Aktivierung des Enzyms wird dieses mit dem Tris/HCl Puffer auf eine Konzentration von 1,67 μg/mL verdünnt.

Zur Messung der Enzymaktivität werden 10 μL Enzymlösung mit 10 μL einer 3%igen (v/v) gepufferten Dimethylsulfoxid-Lösung (Reaktion 1) für 15 Minuten inkubiert. Zur Messung der Enzyminhibitoraktivität werden 10 μL Enzymlösung mit 10 μL einer 3%igen (v/v) gepufferten Dimethylsulfoxid-Lösung, die den Enzyminhibitor enthält, inkubiert (Reaktion 2).

Sowohl bei Reaktion 1 als auch bei Reaktion 2 wird nach Zugabe von 10 μL einer 3%igen (v/v) wässrigen Dimethylsulfoxid-Lösung, die 0,75 mmol/L des Substrates enthält, die Enzymreaktion fluoreszenzspektroskopisch verfolgt (328 nm (Extinktion) / 393 nm(Emission)). Die Enzymaktivität wird dargestellt als Extinktionszunahme/Minute.
Die Inhibitorwirkung wird als prozentuale Hemmung nach folgender Formel berechnet:

$$\% \text{ Hemmung} = 100 - [(\text{Extinktionszunahme/Minute in Reaktion 2}) / (\text{Extinktionszunahme/Minute in Reaktion 1}) \times 100].$$

Der $IC_{50}$, d.h. die für eine 50%ige Hemmung der Enzymaktivität erforderliche Inhibitorkonzentration wird grafisch durch Auftragen der prozentualen Hemmungen bei verschiedenen Inhibitorkonzentrationen ermittelt.

Die Pufferlösung enthält 0,05% Brij (Sigma, Deisenhofen, Deutschland) sowie
0,1 mol/L Tris/HCl, 0,1 mol/L NaCl, 0,01 mol/L $CaCl_2$ (pH=7,5).
Die Enzymlösung enthält 1,67 μg/mL der Enzymdomäne.

Die Substratlösung enthält 0,75 mmol/L des fluorogenen Substrates (7-Methoxycoumarin-4-yl)acetyl-Pro-Leu-Gly-Leu-3-(2',4'-dinitrophenyl)-L-2,3-diaminopropionyl-Ala-Arg-NH2 (Bachem, Heidelberg, Deutschland).

Die nachfolgende Tabelle 2 zeigt die Ergebnisse.

Tabelle 2

| Beispiel Nr. | MMP-3 $IC_{50}$ (nM) | MMP-8 $IC_{50}$ (nM) | MMP-13 $IC_{50}$ (nM) |
|---|---|---|---|
| 13 | 200 | 200 | 700 |
| 21 | 160 | 4 | 100 |
| 29 | >10000 | 10000 | 260 |

(fortgesetzt)

| Beispiel Nr. | MMP-3 $IC_{50}$ (nM) | MMP-8 $IC_{50}$ (nM) | MMP-13 $IC_{50}$ (nM) |
|---|---|---|---|
| 57 | 330 | 320 | 200 |
| 61 | 22 | 3 | 2 |
| 63 | 200 | 3700 | 4,2 |
| 64 | 100 | 20 | 3 |
| 65 | 300 | 100 | 220 |

## Patentansprüche

**1.** Verbindung der Formel I

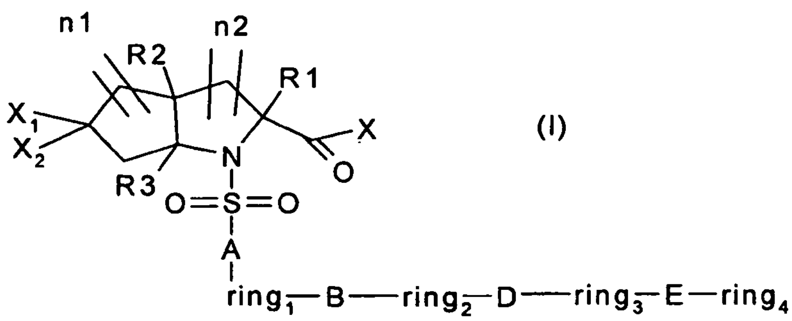

(I)

und/oder alle stereoisomeren Formen der Verbindung der Formel I und/oder Gemische diese Formen in jedem Verhältnis, und/oder ein physiologisch verträgliches Salz der Verbindung der Formel I, wobei

A für -($C_0$-$C_4$)-Alkylen steht,
B, D und E gleich oder verschieden sind und unabhängig voneinander für

1. -($C_0$-$C_4$)-Alkylen,
2. -($C_2$-$C_4$)-Alkenylen,
3. -$S(O)_o$-, wobei o die ganzen Zahlen Null, 1 oder 2 bedeutet,
4. -NH-,
5. -NH-C(O)-,
6. -C(O)-NH-,
7. -NH-$SO_2$-,
8. -NH-C(O)-NH-,
9. -NH-C(S)-,
10. -NH-C(O)-O-,
11. -O-,
12. -O-C(O)-NH-,
13. -C(O)-,
14. -O-$(CH_2)_n$-O-, worin n die ganze Zahl 2 oder 3 bedeutet, oder
15. -O-$(CH_2)_m$-NH-, worin m die ganze Zahl 2 oder 3 bedeutet, stehen,

ring1, ring2 oder ring3 gleich oder verschieden sind und unabhängig voneinander für

1. kovalente Bindung,
2. -($C_6$-$C_{14}$)-Aryl, worin Aryl unsubstituiert oder unabhängig voneinander ein-, zwei- oder dreifach durch G substituiert ist, oder
3. 5- oder 6-gliedriger aromatischer Heteroarylring stehen, worin der Heteroarylring unsubstituiert oder unabhängig voneinander ein-, zwei- oder dreifach durch G substituiert ist,

ring4 für

1. $-(C_6-C_{14})$-Aryl, worin Aryl unsubstituiert oder unabhängig voneinander ein-, zwei- oder dreifach durch G substituiert ist,
2. 5- oder 6-gliedriger aromatischer Heteroarylring, worin der Heteroarylring unsubstituiert oder unabhängig voneinander ein-, zwei- oder dreifach durch G substituiert ist,
3. Heteroaryl, worin Heteroaryl unsubstituiert oder unabhängig voneinander ein-, zwei- oder dreifach durch G substituiert ist, oder
4. für einen der folgenden Reste

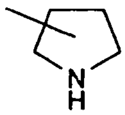
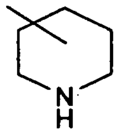
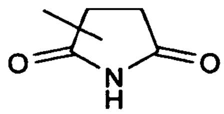
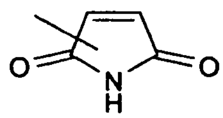
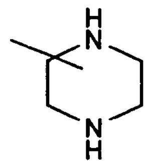
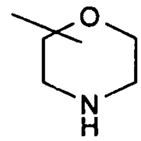

und diese Reste unsubstituiert oder unabhängig voneinander ein-, zwei- oder dreifach durch G substituiert sind, steht,

G für

1. Wasserstoffatom,
2. Halogen,
3. R4 steht und R4

    a) Wasserstoffatom,
    b) $-(C_1-C_6)$-Alkyl, worin Alkyl unsubstituiert oder ein-, zwei- oder dreifach durch Halogen, $-(C_3-C_6)$-Cycloalkyl, $-(C_6-C_{14})$-Aryl oder Heteroaryl substituiert ist,
    c) $-(C_6-C_{14})$-Aryl,
    d) Heteroaryl,
    e) -C(O)-O-R5, worin R5

        e)1) $-(C_1-C_6)$-Alkyl, worin Alkyl unsubstituiert oder ein- oder zweifach durch $-(C_3-C_6)$-Cycloalkyl, $-(C_6-C_{14})$-Aryl, oder Heteroaryl substituiert ist, oder
        e)2) $-(C_6-C_{14})$-Aryl oder Heteroaryl bedeutet,

    f) -C(S)-O-R5, worin R5 wie oben definiert ist,
    g) -C(O)-NH-R6, worin R6

        g)1) $-(C_1-C_6)$-Alkyl, worin Alkyl unsubstituiert oder ein- oder zweifach durch $-(C_3-C_6)$-Cycloalkyl, $-(C_6-C_{14})$-Aryl oder Heteroaryl substituiert ist, oder
        g)2) $-(C_6-C_{14})$-Aryl oder Heteroaryl bedeutet, oder

    h) -C(S)-NH-R6, worin R6 wie oben definiert ist, bedeutet,

4. -O-R4, worin R4 wie oben definiert ist,
5. -C(O)-R5, worin R5 wie oben definiert ist,
6. $-S(O)_p$-R4, worin R4 wie oben definiert ist und p die ganzen Zahlen Null, 1 oder 2 bedeutet,
7. $-NO_2$,
8. -CN oder
9. -N(R3)-R4, worin R3

    9.1) Wasserstoffatom oder
    9.2) $-(C_1-C_6)$-Alkyl bedeutet und R4 wie oben definiert ist, steht,

X für -OH oder -NH-OH steht,
$X_1$ und $X_2$ gleich oder verschieden sind und unabhängig voneinander Wasserstoffatom oder $-(C_1-C_6)$-Alkyl

bedeuten, oder zusammen den Rest =O bilden,
n1 und n2 gleich oder verschieden sind und unabhängig voneinander Null, 1 oder 2 bedeuten,
R1 für

1. Wasserstoffatom oder
2. -($C_1$-$C_6$)-Alkyl, worin Alkyl unsubstituiert oder ein- oder zweifach durch -($C_3$-$C_6$)-Cycloalkyl, -($C_6$-$C_{14}$)-Aryl, oder Heteroaryl substituiert ist, steht,

R2 und R3 gleich oder verschieden sind und unabhängig voneinander für Wasserstoffatom oder -($C_1$-$C_6$)-Alkyl stehen,

wobei die Verbindung 1-(Toluol-4-sulfonyl)-octahydro-indol-2-carbonsäure ausgeschlossen ist.

**2.** Verbindung der Formel I gemäß Anspruch 1, wobei

A für -($C_0$-$C_4$)-Alkylen steht,
B, D und E gleich oder verschieden sind und unabhängig voneinander für

1. -($C_0$-$C_4$)-Alkylen,
2. -($C_2$-$C_4$)-Alkenylen,
3. -S(O)$_o$-, wobei o die ganzen Zahlen Null, 1 oder 2 bedeutet,
4. -NH-,
5. -NH-C(O)-,
6. -C(O)-NH-,
7. -NH-$SO_2$-,
8. -NH-C(O)-NH-,
10. -NH-C(S)-,
10. -NH-C(O)-O-,
11. -O-,
12. -O-C(O)-NH-,
13. -C(O)-,
14. -O-$(CH_2)_n$-O-, worin n die ganze Zahl 2 oder 3 bedeutet, oder
15. -O-$(CH_2)_m$-NH-, worin m die ganze Zahl 2 oder 3 bedeutet, stehen,

ring1, ring2 oder ring3 gleich oder verschieden sind und unabhängig voneinander für

1. kovalente Bindung,
2. -($C_6$-$C_{14}$)-Aryl, worin Aryl ein Rest aus der Reihe Phenyl, Naphthyl, 1-Naphthyl, 2-Naphthyl, Biphenylyl, 2-Biphenylyl, 3-Biphenylyl, 4-Biphenylyl, Anthryl oder Fluorenyl bedeutet und unsubstituiert oder unabhängig voneinander ein-, zwei- oder dreifach durch G substituiert sind, stehen, oder
3. 5- oder 6-gliedriger aromatischer Heteroarylring stehen, worin der Heteroarylring ein Rest aus der Reihe Dihydrofuranyl, Dioxolyl, Dioxanyl, Furanyl, Imidazolidinyl, Imidazolinyl, Imidazolyl, Isoxazolyl, Isoxazolidinyl, 2-Isoxazolinyl, Isothiazolyl, Isothiazolidinyl, 2-Isothiazolinyl, Morpholinyl, Oxazolyl, Oxothiolanyl, Piperazinyl, Piperidinyl, Pyranyl, Pyrazinyl, Pyrazolyl, Pyrazolidinyl, Pyrazolinyl, Pyridazinyl, Pyridinyl, Pyrimidinyl, Pyrrolyl, Pyrrolidinyl, Tetrahydrofuranyl, Tetrahydropyridinyl, Thiazolyl, Thiomorpholinyl, Thiophenyl oder Thiopyranyl bedeutet und unsubstituiert oder unabhängig voneinander ein-, zwei- oder dreifach durch G substituiert sind,

ring4 für

1. -($C_6$-$C_{14}$)-Aryl steht, worin Aryl ein Rest aus der Reihe Phenyl, Naphthyl, 1-Naphthyl, 2-Naphthyl, Biphenylyl, 2-Biphenylyl, 3-Biphenylyl, 4-Biphenylyl, Anthryl oder Fluorenyl bedeutet und unsubstituiert oder unabhängig voneinander ein-, zwei- oder dreifach durch G substituiert sind,
2. 5- oder 6-gliedriger aromatischer Heteroarylring steht, worin der Heteroarylring ein Rest aus der Reihe Dihydrofuranyl, Dioxolyl, Dioxanyl, Furanyl, Imidazolidinyl, Imidazolinyl, Imidazolyl, Isoxazolyl, Isoxazolidinyl, 2-Isoxazolinyl, Isothiazolyyl, Isothiazolidinyl, 2-Isothiazolinyl, Morpholinyl, Oxazolyl, Oxothiolanyl, Piperazinyl, Piperidinyl, Pyranyl, Pyrazinyl, Pyrazolyl, Pyrazolidinyl, Pyrazolinyl, Pyridazinyl, Pyridinyl, Pyrimidinyl, Pyrrolyl, Pyrrolidinyl, Tetrahydrofuranyl, Tetrahydropyridinyl, Thiazolyl, Thiomorpholinyl, Thiophenyl

oder Thiopyranyl bedeutet und unsubstituiert oder unabhängig voneinander ein-, zwei- oder dreifach durch G substituiert sind,

3. Heteroaryl steht, worin Heteroaryl ein Rest aus der Reihe Acridinyl, Azetidinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chromanyl, Chromenyl, Cinnolinyl, Deca-hydrochinolinyl, 2H, 6H-1,5,2-Dithiazinyl, Dihydrofuran[2,3-b]-tetrahydrofuran, Fuaranyl, Furazanyl, Imidazolidihyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridinyl, Pyridyl, Pyrimidinyl, Pyrrolidinyl, Pyrrolinyl, 2H-Pyrrolyl, Pyrrolyl, Chinazolinyl, Chinolinyl, 4H-Chinolizinyl, Chinoxalinyl, Chinuclidinyl, Tetrahydrofuranyl, Tetrahydroisochinolinyl, Tetrahydrochinolinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Thiophenyl, Triazinyl, 1,2,3-Triazolyl, 1,2,3-Triazolyi, 1,2,4-Triazolyi, 1,2,5-Triazolyi, 1,3,4-Triazolyl und Xanthenyl bedeutet und unsubstituiert oder unabhängig voneinander ein-, zwei- oder dreifach durch G substituiert sind, oder

4. für einen der folgenden Reste

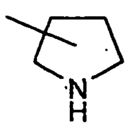 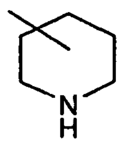 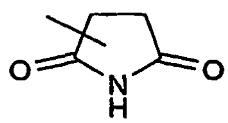 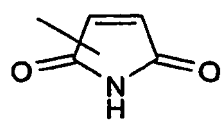 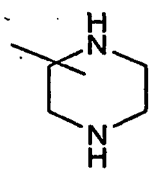 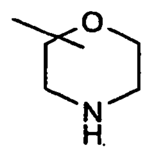

und diese Reste unsubstituiert oder unabhängig voneinander ein-, zwei- oder dreifach durch G substituiert sind, steht,

G für

1. Wasserstoffatom,
2. Halogen,
3. R4 steht und R4

a) Wasserstoffatom,
b) -($C_1$-$C_6$)-Alkyl, worin Alkyl unsubstituiert oder ein-, zwei- oder dreifach durch Halogen, -($C_3$-$C_6$)-Cycloalkyl, Phenyl, Naphthyl oder Heteroaryl, wobei Heteroaryl wie oben definiert, substituiert ist,
c) Phenyl oder Naphthyl,
d) Heteroaryl, wobei Heteroaryl wie oben definiert ist,
e) -C(O)-O-R5, worin R5

e)1) -($C_1$-$C_6$)-Alkyl, worin Alkyl unsubstituiert oder ein- oder zweifach durch -($C_3$-$C_6$)-Cycloalkyl, Phenyl, Naphthyl oder Heteroaryl, wobei Heteroaryl wie oben definiert ist, substituiert ist,
e)2) Phenyl oder Naphthyl oder
e)3) Heteroaryl, wobei Heteroaryl wie oben definiert, substituiert ist, bedeutet,

f) -C(S)-O-R5, worin R5 wie oben definiert ist,
g) -C(O)-NH-R6, worin R6

g)1) -($C_1$-$C_6$)-Alkyl, worin Alkyl unsubstituiert oder ein- oder zweifach durch -($C_3$-$C_6$)-Cycloalkyl, Phenyl, Naphthyl oder Heteroaryl, wobei Heteroaryl wie oben definiert ist, substituiert ist,
g)2) Phenyl oder Naphthyl oder
g)3) Heteroaryl, wobei Heteroaryl wie oben definiert, substituiert ist, bedeutet, oder

h) -C(S)-NH-R6, worin R6 wie oben definiert ist, bedeutet,

4. -0-R4, worin R4 wie oben definiert ist,
5. -C(O)-R5, worin R5 wie oben definiert ist,
6. $-S(O)_p-R4$, worin R4 wie oben definiert ist und p die ganzen Zahlen Null, 1 oder 2 bedeutet,
7. $-NO_2$,
8. -CN oder
9. -N(R3)-R4, worin R3

9.1) Wasserstoffatom oder
9.2) $-(C_1-C_6)$-Alkyl bedeutet und R4 wie oben definiert ist, steht,

X für -OH oder -NH-OH steht,
$X_1$ und $X_2$ gleich oder verschieden sind und unabhängig voneinander Wasserstoffatom oder $-(C_1-C_6)$-Alkyl bedeuten, oder zusammen den Rest =0 bilden,
n1 und n2 gleich oder verschieden sind und unabhängig voneinander Null, 1 oder 2 bedeuten,
R1 für

1. Wasserstoffatom oder
2. $-(C_1-C_6)$-Alkyl, worin Alkyl unsubstituiert oder ein- oder zweifach durch $-(C_3-C_6)$-Cycloalkyl, Phenyl, Naphthyl oder Heteroaryl, wobei Heteroaryl wie oben definiert ist, substituiert ist, steht,

R2 und R3 gleich oder verschieden sind und unabhängig voneinander für Wasserstoffatom oder $-(C_1-C_6)$-Alkyl stehen.

**3.** Verbindung der Formel I gemäß der Ansprüche 1 oder 2, wobei

A für $-(C_0-C_4)$-Alkylen steht,
B, D und E gleich oder verschieden sind und unabhängig voneinander für

1. $-(C_0-C_2)$-Alkylen,
2. $-C_2$-Alkenylen,
3. $-S(O)_o-$, wobei o die ganzen Zahl 2 bedeutet,
4. -NH-,
5. -NH-C(O)-,
6. -C(O)-NH-,
7. -NH-C(O)-NH-,
8. -0- oder
9. -C(O)-, stehen,

ring1, ring2 oder ring3 gleich oder verschieden sind und unabhängig voneinander für

1. kovalente Bindung stehen,
2. Phenyl oder Naphthyl bedeutet und unsubstituiert oder unabhängig voneinander ein-, zwei- oder dreifach durch G substituiert sind, oder
3. 5- oder 6-gliedriger aromatischer Heteroarylring stehen, worin der Heteroarylring ein Rest aus der Reihe Dihydrofuranyl, Furanyl, Morpholinyl, Piperazinyl, Piperidinyl, Pyridinyl, Pyrimidinyl, .Pyrrolyl, Thiazolyl oder Thiophenyl bedeutet und unsubstituiert oder unabhängig voneinander ein-, zwei- oder dreifach durch G substituiert sind,

ring4 für

1. Phenyl oder Naphthyl steht und unsubstituiert oder unabhängig voneinander ein-, zwei- oder dreifach durch G substituiert ist,
2. 5- oder 6-gliedriger aromatischer Heteroarylring steht, worin der Heteroarylring ein Rest aus der Reihe Dihydrofuranyl, Furanyl, Morpholinyl, Piperazinyl, Piperidinyl, Pyridinyl, Pyrimidinyl, Pyrrolyl, Thiazolyl oder Thiophenyl bedeutet und bedeutet und unsubstituiert oder unabhängig voneinander ein-, zwei- oder dreifach durch G substituiert ist,

3. Heteroaryl steht, worin Heteroaryl ein Rest aus der Reihe Benzofuranyl, Benzothiophenyl, Dihydrofuranyl, Furanyl, Morpholinyl, Piperazinyl, Piperidinyl, Pyridinyl, Pyridothiophenyl, Pyrimidinyl, Pyrrolyl, Thiazolyl oder Thiophenyl bedeutet und unsubstituiert oder unabhängig voneinander ein-, zwei- oder dreifach durch G substituiert sind, oder
4. für einen der folgenden Reste

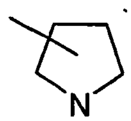
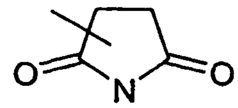

und diese Reste unsubstituiert oder unabhängig voneinander ein-, zwei- oder dreifach durch G substituiert sind, steht,

G für

1. Wasserstoffatom,
2. Br; Cl, I oder F,
3. R4 steht und R4

a) Wasserstoffatom,
b) -($C_1$-$C_4$)-Alkyl, worin Alkyl unsubstituiert oder ein-, zwei- oder dreifach durch Br, Cl, F, -$C_3$-Cycloalkyl, Phenyl, Naphthyl oder Heteroaryl, wobei Heteroaryl wie oben definiert, substituiert ist,
c) Phenyl oder Naphthyl,
d) Heteroaryl, wobei Heteroaryl wie oben definiert ist,
e) -C(O)-O-R5, worin R5

e)1) -($C_1$-$C_4$)-Alkyl, worin Alkyl unsubstituiert oder ein- oder zweifach durch -$C_3$-Cycloalkyl, Phenyl, Naphthyl oder Heteroaryl, wobei Heteroaryl wie oben definiert ist, substituiert ist,
e)2) Phenyl oder Naphthyl oder
e)3) Heteroaryl, wobei Heteroaryl wie oben definiert, substituiert ist, bedeutet, oder

f) -C(O)-NH-R6, worin R6 für

f)1) -($C_1$-$C_4$)-Alkyl, worin Alkyl unsubstituiert oder ein- oder zweifach durch -$C_3$-Cycloalkyl, Phenyl, Naphthyl oder Heteroaryl, wobei Heteroaryl wie oben definiert ist, substituiert ist,
f)2) Phenyl oder Naphthyl oder
f)3) Heteroaryl, wobei Heteroaryl wie oben definiert, substituiert ist, steht, bedeutet,

4. -O-R4, worin R4 wie oben definiert ist,
5. -C(O)-R5, worin R5 wie oben definiert ist,
6. -$S(O)_p$-R4, worin R4 wie oben definiert ist und p die ganze Zahl 2 bedeutet,
7. -$NO_2$,
8. -CN oder
9. -N(R3)-R4, worin R3

9.1) Wasserstoffatom oder
9.2) -($C_1$-$C_6$)-Alkyl bedeutet und R4 wie oben definiert ist, steht,

X für -OH oder -NH-OH steht,
$X_1$ und $X_2$ gleich sind und Wasserstoffatom bedeuten,
n1 und n2 gleich sind und 1 bedeuten, oder ungleich sind und n1 gleich 2 und n2 gleich 1 bedeuten,
R1 für Wasserstoffatom steht, und
R2 und R3 gleich sind und für Wasserstoffatom stehen.

**4.** Verfahren zur Herstellung der Verbindung der Formel I gemäß einem oder mehreren der Ansprüche 1 bis 3 und/oder einer stereoisomeren Form der Verbindung der Formel I und/oder eines physiologisch verträglichen Salzes

der Verbindung der Formel I, das **dadurch gekennzeichnet ist, dass** man

a) eine Verbindung der Formel IV,

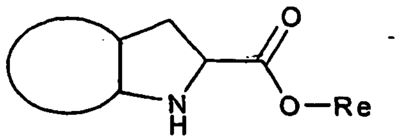

(IV)

worin Re ein Wasserstoffatom oder eine Ester-Schutzgruppe darstellt, mit einer Verbindung der Formel V, worin A, B, D, E und ring1, ring2, ring3, ring4 wie in Formel I definiert sind, und worin Rz Chloratom, Imidazoyl oder OH bedeutet, in Gegenwart einer Base oder nach Silylierung mit einem geeigneten

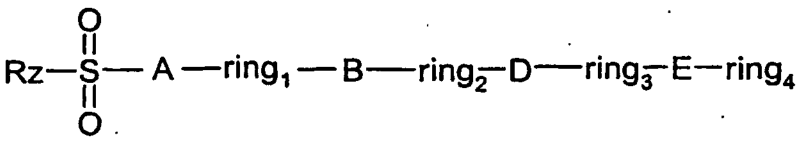

(V)

Silylierungsmittel zu einer Verbindung der Formel VI umsetzt,

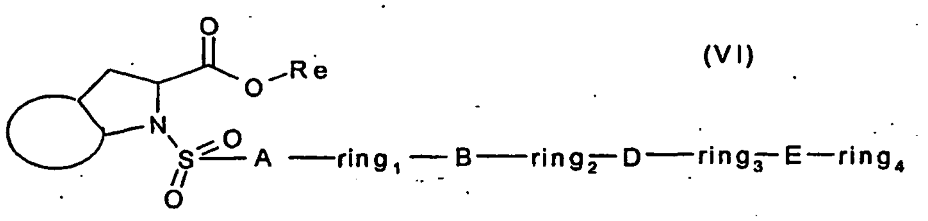

(VI)

worin A, B, D, E, Re und ring1, ring2, ring3 und ring4 wie oben definiert sind,

b) für den Fall Re = Ester eine nach a) hergestellte Verbindung der Formel VI mit einer Alkalilauge wie NaOH oder LiOH und anschließender Säurebehandlung zu der erfindungsgemäßen Carbonsäure der Formel I umsetzt, wobei gegebenenfalls vorher noch Modifikationen in einer der Seitenketten der Ringe ring1-ring4 vorgenommen wurden

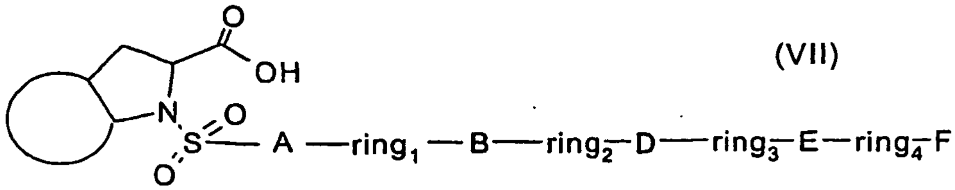

(VII)

und anschließend diese in die erfindungsgemäße Hydroxamsäure, wobei X = NH-OH bedeutet, der Formel umwandeln,

c) eine nach Verfahren a) oder b) hergestellte Verbindung der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder

d) die nach den Verfahren b) oder c) hergestellte Verbindung der Formel I entweder in freier Form isoliert oder im Falle des Vorliegens von sauren oder basischen Gruppen in physiologisch verträgliche Salze umwandelt.

**5.** Arzneimittel, **gekennzeichnet durch** einen wirksamen Gehalt von mindestens einer Verbindung der Formel I gemäß einem oder mehreren der Ansprüche 1 bis 3 zusammen mit einem pharmazeutisch geeigneten und physiologisch verträglichen Trägerstoff, Zusatzstoff und/oder anderen Wirk- und Hilfsstoffen.

**6.** Verwendung der Verbindung der Formel I gemäß einem oder mehreren der Ansprüche 1 bis 3 zur Herstellung eines Arzneimittels zur Prophylaxe und Therapie von Erkrankungen wie Osteoarthrosen, Spondylosen, Knorpelschwund nach Gelenktrauma oder längerer Gelenksruhigstellung nach Meniskus- oder Patellaverletzungen oder Bänderrissen, Erkrankungen des Bindegewebes wie Kollagenosen, Periodontalerkrankungen, Wundheilungsstörungen und chronische Erkrankungen des Bewegungsapparates wie entzündliche, immunologisch oder stoffwechselbedingte akute und chronische Arthritiden, Arthropathien, Myalgien und Störungen des Knochenstoffwechsels, zur Behandlung der Ulceration, Atherosklerose und Stenosen, Behandlung von Entzündungen, Krebserkrankungen, Tumormetastasenbildung, Kachexie, Anorexie, Herzversagen und septischem Schock oder Prophylaxe von Myocard- und Cerebral-Infarkten.

## Claims

**1.** A compound of formula I

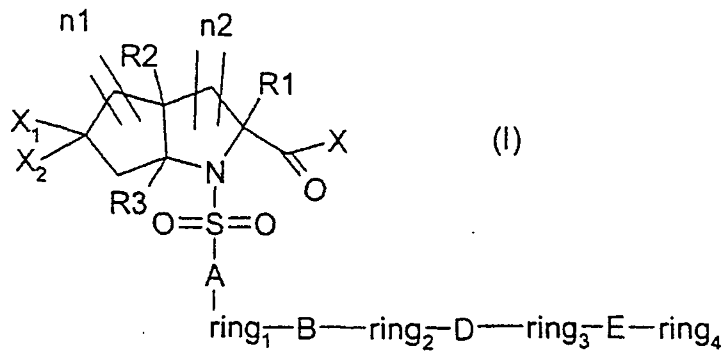

(I)

and/or all the stereoisomeric forms of the compound of formula I and/or mixtures of these forms in any ratio, and/or a physiologically tolerated salt of the compound of formula I, wherein

A is -($C_0$-$C_4$)-alkylene,
B, D and E are identical or different and are, independently of each other,

1. -($C_0$-$C_4$)-alkylene,
2. -($C_2$-$C_4$)-alkenylene,
3. -$S(O)_o$-, where o is the integers zero, 1 or 2,
4. -NH-,
5. -NH-C(O)-,
6. -C(O)-NH-,
7. -NH-$SO_2$-,
8. -NH-C(O)-NH-,
9. -NH-C(S)-,
10. -NH-C(O)-O-,
11. -O-,
12. -O-C(O)-NH-,
13. -C(O)-,
14. -O-$(CH_2)_n$-O-, in which n is the integer 2 or 3, or
15. -O-$(CH_2)_m$-NH-, in which m is the integer 2 or 3,

ring 1, ring 2 or ring 3 are identical or different and are, independently of each other,

1. covalent bond,
2. -($C_6$-$C_{14}$)-aryl, in which aryl is unsubstituted or substituted, independently of each other, once, twice or

three times, by G, or
3. 5- or 6-membered aromatic heteroaryl ring, in which the heteroaryl ring is unsubstituted or substituted, independently of each other, once, twice or three times, by G,

ring 4 is

1. -($C_6$-$C_{14}$)-aryl, in which aryl is unsubstituted or substituted, independently of each other, once, twice or three times, by G,
2. 5- or 6-membered aromatic heteroaryl ring, in which the heteroaryl ring is unsubstituted or substituted, independently of each other, once, twice or three times, by G,
3. heteroaryl, in which heteroaryl is unsubstituted or substituted, independently of each other, once, twice or three times, by G, or
4. one of the following radicals

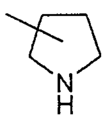 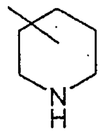 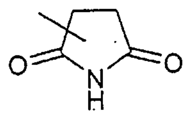 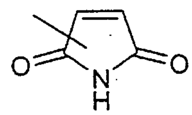 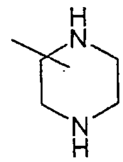 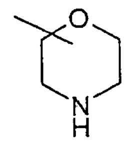

and these radicals are unsubstituted or substituted, independently of each other, once, twice or three times, by G,

G is

1. hydrogen atom,
2. halogen,
3. R4 and R4 is

a) hydrogen atom,
b) -($C_1$-$C_6$)-alkyl, in which alkyl is unsubstituted or substituted, once, twice or three times, by halogen, -($C_3$-$C_6$)-cycloalkyl, -($C_6$-$C_{14}$)-aryl or heteroaryl,
c) -($C_6$-$C_{14}$)-aryl,
d) heteroaryl,
e) -C(O)-O-R5, in which R5 is

e)1) -($C_1$-$C_6$)-alkyl, in which alkyl is unsubstituted or substituted, once or twice, by -($C_3$-$C_6$)-cycloalkyl, -($C_6$-$C_{14}$)-aryl, or heteroaryl, or
e)2) -($C_6$-$C_{14}$)-aryl or heteroaryl,

f) -C(S)-O-R5, in which R5 is defined as above,
g) -C(O)-NH-R6, in which R6 is

g)1) -($C_1$-$C_6$)-alkyl, in which alkyl is unsubstituted or substituted, once or twice, by -($C_3$-$C_6$)-cycloalkyl, -($C_6$-$C_{14}$)-aryl or heteroaryl, or
g)2) -($C_6$-$C_{14}$)-aryl or heteroaryl, or

h) -C(S)-NH-R6, in which R6 is defined as above,

4. -O-R4, in which R4 is defined as above,
5. -C(O)-R5, in which R5 is defined as above,
6. -S(O)p-R4, in which R4 is defined as above and p is the integers zero, 1 or 2,
7. -$NO_2$,
8. -CN or
9. -N(R3)-R4, in which R3 is

9.1) hydrogen atom, or
9.2) -($C_1$-$C_6$)-alkyl and R4 is defined as above,

X is -OH or -NH-OH,
$X_1$ and $X_2$ are identical or different and are, independently of each other, hydrogen atom or -($C_1$-$C_6$)-alkyl, or together form the radical =O,
n1 and n2 are identical or different and are, independently of each other, zero, 1 or 2,
R1 is

1. hydrogen atom, or
2. -($C_1$-$C_6$)-alkyl, in which alkyl is unsubstituted or substituted, once or twice, by -($C_3$-$C_6$)-cycloalkyl, -($C_6$-$C_{14}$)-aryl or heteroaryl,

R2 and R3 are identical or different and are, independently of each other, hydrogen atom or -($C_1$-$C_6$)-alkyl,

wherein the compounds 1-(toluene-4-sulfonyl)octahydroindole-2-carboxylic acid and N-[(3,5-dichlorobenzene)sulfonyl)]-(3a(S), 7a(S))octahydroindole-2(S)-carboxylic acid are excluded.

**2.** A compound of formula I as claimed in claim 1, wherein

A is -($C_0$-$C_4$)-alkylene,
B, D and E are identical or different and are, independently of each other,

1. -($C_0$-$C_4$)-alkylene,
2. -($C_2$-$C_4$)-alkylene,
3. -$S(O)_o$-, where o is the integers zero, 1 or 2,
4. -NH-,
5. -NH-C(O)-,
6. -C(O)-NH-,
7. -NH-$SO_2$-,
8. -NH-C(O)-NH-,
9. -NH-C(S)-,
10. -NH-C(O)-O-,
11. -O-,
12. -O-C(O)-NH-,
13. -C(O)-,
14. -O-$(CH_2)_n$-O-, in which n is the integer 2 or 3, or
15. -O-$(CH_2)_m$-NH-, in which m is the integer 2 or 3,

ring 1, ring 2 or ring 3 are identical or different and are, independently of each other,

1. covalent bond,
2. -($C_6$-$C_{14}$)-aryl, in which aryl is a radical from the series phenyl, naphthyl, 1-naphthyl, 2-naphthyl, biphenylyl, 2-biphenylyl, 3-biphenylyl, 4-biphenylyl, anthryl or fluorenyl and are unsubstituted or substituted, independently of each other, once, twice or three times, by G, or
3. 5- or 6-membered aromatic heteroaryl ring, in which the heteroaryl ring is a radical from the series dihydrofuranyl, dioxolyl, dioxanyl, furanyl, imidazolidinyl, imidazolinyl, imidazolyl, isoxazolyl, isoxazolidinyl, 2-isoxazolinyl, isothiazolyl, isothiazolidinyl, 2-isothiazolinyl, morpholinyl, oxazolyl, oxothiolanyl, piperazinyl, piperidinyl, pyranyl, pyrazinyl, pyrazolyl, pyrazolidinyl, pyrazolinyl, pyridazinyl, pyridinyl, pyrimidinyl, pyrrolyl, pyrrolidinyl, tetrahydrofuranyl, tetrahydropyridinyl, thiazolyl, thiomorpholinyl, thiophenyl or thiopyranyl and are unsubstituted or substituted, independently of each other, once, twice or three times, by G,

ring 4 is

1. -($C_6$-$C_{14}$)-aryl, in which aryl is a radical from the series phenyl, naphthyl, 1-naphthyl, 2-naphthyl, biphenylyl, 2-biphenylyl, 3-biphenylyl, 4-biphenylyl, anthryl or fluorenyl and are unsubstituted or substituted, independently of each other, once, twice or three times, by G,
2. 5- or 6-membered aromatic heteroaryl ring, in which the heteroaryl ring is a radical from the series

dihydrofuranyl, dioxolyl, dioxanyl, furanyl, imidazolidinyl, imidazolinyl, imidazolyl, isoxazolyl, isoxazolidinyl, 2-isoxazolinyl, isothiazolyl, isothiazolidinyl, 2-isothiazolinyl, morpholinyl, oxazolyl, oxothiolanyl, piperazinyl, piperidinyl, pyranyl, pyrazinyl, pyrazolyl, pyrazolidinyl, pyrazolinyl, pyridazinyl, pyridinyl, pyrimidinyl, pyrrolyl, pyrrolidinyl, tetrahydrofuranyl, tetrahydropyridinyl, thiazolyl, thiomorpholinyl, thiophenyl or thiopyranyl and are unsubstituted or substituted, independently of each other, once, twice or three times, by G,

3. heteroaryl, in which heteroaryl is a radical from the series acridinyl, azetidinyl, benzimidazolyl, benzofuranyl, benzothiofuranyl, benzothiophenyl, benzoxazolyl, benzothiazolyl, benzotriazolyl, benzotetrazolyl, benzisoxazolyl, benzisothiazolyl, benzimidazalinyl, carbazolyl, 4aH-carbazolyl, carbolinyl, chromanyl, chromenyl, cinnolinyl, deca-hydroquinolinyl, 2H,6H-1,5,2-dithiazinyl, dihydrofuran-[2,3-b]tetrahydrofuran, fuaranyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl (benzimidazolyl), isothiazolyl, isoxazolyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolidinyl, pyrimidinyl, phenanthridinyl, phenanthrolinyl, phenazinyl, phenothiazinyl, phenoxathiinyl, phenoxazinyl, phthalazinyl, piperazinyl, piperidinyl, pteridinyl, purynyl, pyranyl, pyrazinyl, pyroazolidinyl, pyrazolinyl, pyrazolyl, pyridazinyl, pryidooxazolyl, pyridoimidazolyl, pyridothiazolyl, pyridothiophenyl, pyridinyl, pyridyl, pyrimidinyl, pyrrolidinyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisoquinolinyl, tetrahydroquinolinyl, 6H-1,2,5-thiadazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl, triazinyl, 1,2,3-triazolyl, 1,2,4-triazolyl, 1,2,5-triazolyl, 1,3,4-triazolyl and xanthenyl, and are unsubstituted or substituted, independently of each other, once, twice or three times, by G, or

4. is one of the following radicals

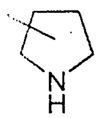 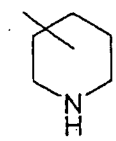 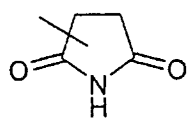 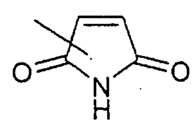 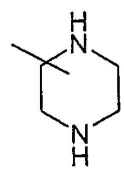 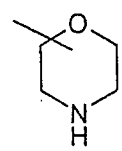

and these radicals are unsubstituted or substituted, independently of each other, once, twice or three times, by G,

G is

1. hydrogen atom,
2. halogen,
3. R4, and R4 is

   a) hydrogen atom,
   b) -($C_1$-$C_6$)-alkyl, in which alkyl is unsubstituted or substituted, once, twice or three times, by halogen, -($C_3$-$C_6$)-cycloalkyl, phenyl, naphthyl or heteroaryl, where heteroaryl is defined as above,
   c) phenyl or naphthyl,
   d) heteroaryl, where heteroaryl is defined as above,
   e) -C(O)-O-R5, in which R5 is

      e)1) -($C_1$-$C_6$)-alkyl, in which alkyl is unsubstituted or substituted, once or twice, by -($C_3$-$C_6$)-cycloalkyl, phenyl, naphthyl or heteroaryl, where heteroaryl is as defined above,
      e)2) phenyl or naphthyl, or
      e)3) heteroaryl, where heteroaryl is as defined above,

   f) -C(S)-O-R5, in which R5 is defined as above,
   g) -C(O)-NH-R6, in which R6 is

      g)1) -($C_1$-$C_6$)-alkyl, in which alkyl is unsubstituted or substituted, once or twice, by -($C_3$-$C_6$)-cycloalkyl, phenyl, naphthyl or heteroaryl, where heteroaryl is defined as above,

g)2) phenyl or naphthyl, or
g)3) heteroaryl, where heteroaryl is as defined above,or

h) -C(S)-NH-R6, in which R6 is defined as above,

4. -O-R4, in which R4 is defined as above,
5. -C(O)-R5, in which R5 is defined as above,
6. -S(O)p-R4, in which R4 is defined as above and p is the integers zero, 1 or 2,
7. -$NO_2$,
8. -CN, or
9. -N(R3)-R4, in which R3 is

9.1) hydrogen atom, or
9.2) -($C_1$-$C_6$)-alkyl and R4 is defined as above,

X is -OH or -NH-OH,
$X_1$ and $X_2$ are identical or different and are, independently of each other, hydrogen atom or -($C_1$-$C_6$)-alkyl, or together form the radical =O,
n1 and n2 are identical or different and are, independently of each other, zero, 1 or 2,
R1 is

1. hydrogen atom, or
2. -($C_1$-$C_6$)-alkyl, in which alkyl is unsubstituted or substituted, once or twice, by -($C_3$-$C_6$)-cycloalkyl, phenyl, naphthyl or heteroaryl, where heteroaryl is defined as above,

R2 and R3 are identical or different and are, independently of each other, hydrogen atom or -($C_1$-$C_6$)-alkyl.

**3.** A compound of formula I as claimed in claim 1 or 2, wherein

A is -($C_0$-$C_4$)-alkylene,
B, D and E are identical or different and are, independently of each other,

1. -($C_0$-$C_2$)-alkylene,
2. -$C_2$-alkenylene,
3. -$S(O)_o$-, where o is the integer 2,
4. -NH-,
5. -NH-C(O)-,
6. -C(O)-NH-,
7. -NH-C(O)-NH-,
8. -O-, or
9. -C(O)-,

ring 1, ring 2 or ring 3 are identical or different and are, independently of each other,

1. covalent bond,
2. phenyl or naphthyl and are unsubstituted or substituted, independently of each other, once, twice or three times, by G, or
3. 5- or 6-membered aromatic heteroaryl ring, in which the heteroaryl ring is a radical from the series dihydrofuranyl, furanyl, morpholinyl, piperazinyl, piperidinyl, pyridinyl, pyrimidinyl, pyrrolyl, thiazolyl or thiophenyl and are unsubstituted or substituted, independently of each other, once, twice or three times, by G,

ring 4 is

1. phenyl or naphthyl and is unsubstituted or substituted, independently of each other, once, twice or three times, by G,
2. 5- or 6-membered aromatic heteroaryl ring, in which the heteroaryl ring is a radical from the series dihydrofuranyl, furanyl, morpholinyl, piperazinyl, piperidinyl, pyridinyl, pyrimidinyl, pyrrolyl, thiazolyl or thiophenyl and is unsubstituted or substituted, independently of each other, once, twice or three times, by G,

3. heteroaryl, in which heteroaryl is a radical from the series benzofuranyl, benzothiophenyl, dihydrofuranyl, furanyl, morpholinyl, piperazinyl, piperidinyl, pyridinyl, pyridothiophenyl, pyrimidinyl, pyrrolyl, thiazolyl or thiophenyl and are unsubstituted or substituted, independently of each other, once, twice or three times, by G, or
4. is one of the following radicals

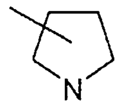

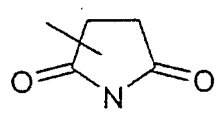

and these radicals are unsubstituted or substituted, independently of each other, once, twice or three times, by G,

G is

1. hydrogen atom,
2. Br; Cl, I or F,
3. R4, and R4 is

a) hydrogen atom,
b) -($C_1$-$C_4$)-alkyl, in which alkyl is unsubstituted or substituted, once, twice or three times, by Br, Cl, F, -$C_3$-cycloalkyl, phenyl, naphthyl or heteroaryl, where heteroaryl is defined as above,
c) phenyl or naphthyl,
d) heteroaryl, where heteroaryl is defined as above,
e) -C(O)-O-R5, in which R5 is

e)1) -($C_1$-$C_4$)-alkyl, in which alkyl is unsubstituted or substituted, once or twice, by -$C_3$-cycloalkyl, phenyl, naphthyl or heteroaryl, where heteroaryl is as defined above,
e)2) phenyl or naphthyl, or
e)3) heteroaryl, where heteroaryl is as defined above, or

f) -C(O)-NH-R6, in which R6 is

f)1) -($C_1$-$C_4$)-alkyl, in which alkyl is unsubstituted or substituted, once or twice, by -$C_3$-cycloalkyl, phenyl, naphthyl or heteroaryl, where heteroaryl is defined as above,
f)2) phenyl or naphthyl, or
f)3) heteroaryl, where heteroaryl is as defined above,

4. -O-R4, in which R4 is defined as above,
5. -C(O)-R5, in which R5 is defined as above,
6. -S(O)p-R4, in which R4 is defined as above and p is the integer 2,
7. -$NO_2$,
8. -CN, or
9. -N(R3)-R4, in which R3 is

9.1) hydrogen atom, or
9.2) -($C_1$-$C_6$)-alkyl and R4 is defined as above,

X is -OH or -NH-OH,
$X_1$ and $X_2$ are identical and are hydrogen atom,
n1 and n2 are identical and are 1 or are nonidentical and n1 is 2 and n2 is 1,
R1 is hydrogen atom, and
R2 and R3 are identical and are hydrogen atom.

**4.** A process for preparing the compound of formula I as claimed in one or more of claims 1 to 3 and/or a stereoisomeric form of the compound of formula I and/or a physiologically tolerated salt of the compound of formula I, which comprises

a) reacting a compound of formula

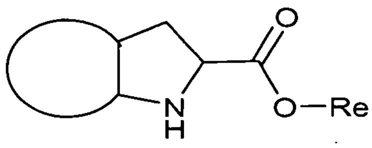

IV, (IV)

in which Re is a hydrogen atom or an ester protecting group, with a compound of formula V,

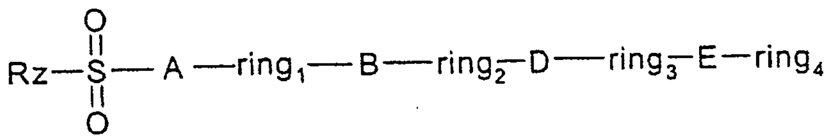

(V)

in which A, B, D, E and ring 1, ring 2, ring 3 and ring 4 are defined as in formula I, and in which Rz is chlorine atom, imidazolyl or OH,

in the presence of a base, or after silylation with a suitable silylating agent, to give a compound of formula VI,

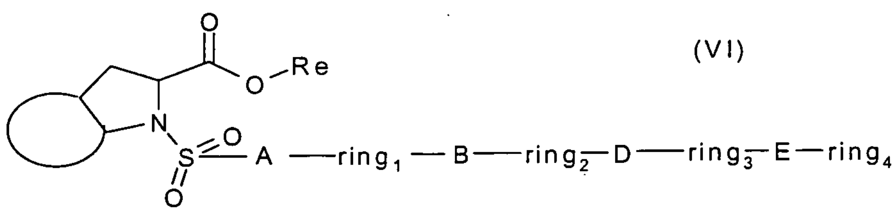

(VI)

in which A, B, D, E, Re and ring 1, ring 2, ring 3 and ring 4 are defined as above,

b) where Re = ester, reacting a compound of formula VI, which has been prepared in accordance with a), with a solution of an alkali such as NaOH or LiOH, and then treating with acid, to give the novel carboxylic acid of formula I, with modifications in one of the side chains of the rings ring 1-ring 4 also having been previously carried out, where appropriate,

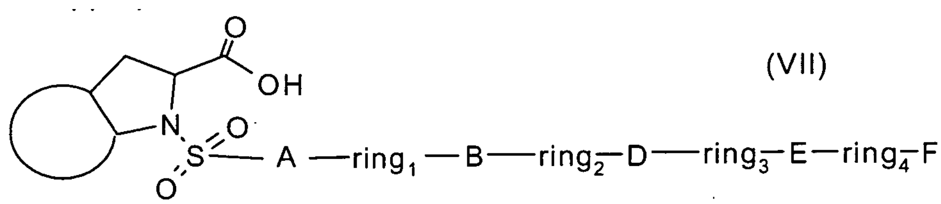

(VII)

and then converting this compound into the novel hydroxamic acid, where X = NH-OH, of formula I,

c) separating a compound of formula I, which has been prepared in accordance with process a) or b) and which arises in enantiomeric forms on account of its chemical structure, into the pure enantiomers by means of salt formation with enantiomerically pure acids or bases, chromatography on chiral stationary phases or derivatization using chiral, enantiomerically pure compounds, such as amino acids, separation of the resulting diastereomers and elimination of the chiral auxiliary groups, or

d) either isolating the compound of formula I, which has been prepared in accordance with processes b) or c), in free form or, when acidic or basic groups are present, converting it into physiologically tolerated salts.

**5.** A pharmaceutical, which comprises an effective content of at least one compound of formula I as claimed in one or more of claims 1 to 3 together with a pharmaceutically suitable and physiologically tolerated carrier substance, additive and/or other active compounds and auxiliary substances.

**6.** The use of the compound of formula I as claimed in one or more of claims 1 to 3 for producing a pharmaceutical for the prophylaxis and therapy of diseases such as osteoarthroses, spondyloses, cartilage loss following joint trauma or a relatively long period of joint immobilization following meniscus or patella injuries or ligament rupture, diseases of the connective tissue such as collagenoses, periodontal diseases, wound healing disturbances and chronic diseases of the locomotory apparatus, such as inflammatory, immunologically-determined or metabolism-determined acute and chronic arthritides, arthropathies, myalgias and disturbances in bone metabolism, for treatment of ulceration, atherosclerosis and stenoses, treatment of inflammations, cancer diseases, tumor metastasis formation, cachexia, anorexia, heart failure and septic shock or prophylaxis of myocardial and cerebral infarcts.

## Revendications

**1.** Composé de formule I

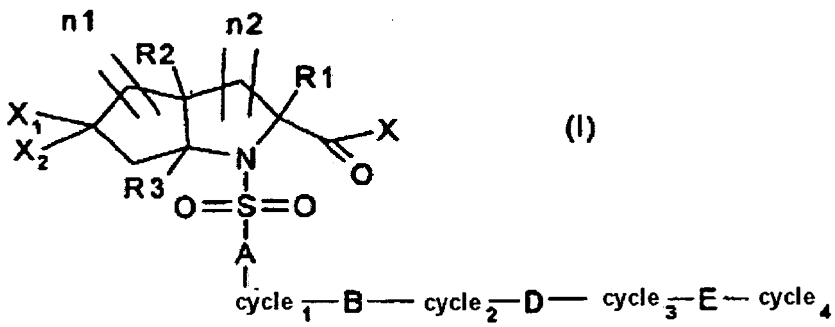

(I)

et/ou toutes les formes stéréo-isomères du composé de formule (I) et/ou les mélanges de ces formes, dans tous les rapports, et/ou un sel physiologiquement acceptable du composé de formule (I), où

A représente -($C_0$-$C_4$)-alkylène,
B, D et E sont identiques ou différents et représentent, indépendamment l'un de l'autre,

1. -($C_0$-$C_4$)-alkylène,
2. -($C_2$-$C_4$)-alcénylène,
3. -$S(O)_o$-, où o signifie les valeurs entières zéro, 1 ou 2,
4. -NH-,
5. -NH-C(O)-,
6. -C(O)-NH-,
7. -NH-$SO_2$-,
8. -NH-C(O)-NH-,
9. -NH-C(S)-,
10. -NH-C(O)-O-,
11. -O-,
12. -O-C(O)-NH-,
13. -C(O)-,
14. -O-$(CH_2)_n$-O-, où n signifie le nombre entier 2 ou 3, ou
15. -O-$(CH_2)_m$-NH-, où m signifie le nombre entier 2 ou 3,

cycle1, cycle2 ou cycle3 sont identiques ou différents et représentent, indépendamment l'un de l'autre,

1. une liaison covalente,
2. -($C_6$-$C_{14}$)-aryle, où aryle est non substitué ou monosubstitué, disubstitué ou trisubstitué, indépendamment l'un de l'autre, par G, ou
3. un cycle hétéroaryle aromatique de 5 ou 6 chaînons, où le cycle hétéroaryle est non substitué ou monosubstitué, disubstitué ou trisubstitué, indépendamment l'un de l'autre, par G,

cycle4 représente

1. -($C_6$-$C_{14}$)-aryle, où aryle est non substitué ou monosubstitué, disubstitué ou trisubstitué, indépendamment l'un de l'autre, par G,
2. un cycle hétéroaryle aromatique de 5 ou 6 chaînons, où le cycle hétéroaryle est non substitué ou monosubstitué, disubstitué ou trisubstitué, indépendamment l'un de l'autre, par G,
3. hétéroaryle, où hétéroaryle est non substitué ou monosubstitué, disubstitué ou trisubstitué, indépendamment l'un de l'autre, par G,
4. un des radicaux suivants

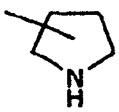 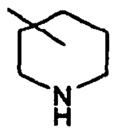 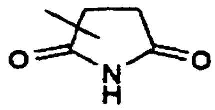 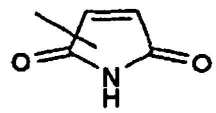 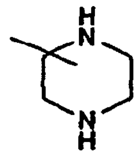 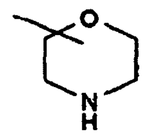

et ces radicaux sont non substitués ou monosubstitués, disubstitués ou trisubstitués, indépendamment l'un de l'autre, par G,

G représente

1. un atome d'hydrogène
2. halogène
3. R4 et R4 signifie

   a) un atome d'hydrogène
   b) -($C_1$-$C_6$)-alkyle, où alkyle est non substitué ou monosubstitué, disubstitué ou trisubstitué par halogène, -($C_3$-$C_6$)-cycloalkyle, -($C_6$-$C_{14}$)-aryle ou hétéroaryle,
   c) -($C_6$-$C_{14}$)-aryle,
   d) hétéroaryle,
   e) -C(O)-O-R5, où R5 signifie

      e)1) -($C_1$-$C_6$)-alkyle, où alkyle est non substitué ou monosubstitué ou disubstitué par -($C_3$-$C_6$)-cycloalkyle, -($C_6$-$C_{14}$)-aryle ou hétéroaryle, ou
      e)2) -($C_6$-$C_{14}$)-aryle ou hétéroaryle,

   f) -C(S)-O-R5, où R5 est défini comme ci-dessus,
   g) -C(O)-NH-R6, où R6 signifie

      g)1) -($C_1$-$C_6$)-alkyle, où alkyle est non substitué ou monosubstitué ou disubstitué par -($C_3$-$C_6$)-cycloalkyle, -($C_6$-$C_{14}$)-aryle ou hétéroaryle, ou
      g)2) -($C_6$-$C_{14}$)-aryle ou hétéroaryle, ou

   h) -C(S)-NH-R6, où R6 est défini comme ci-dessus,

4. -O-R4, où R4 est défini comme ci-dessus,
5. -C(O)-R5, où R5 est défini comme ci-dessus,
6. -$S(O)_p$-R4, où R4 est défini comme ci-dessus et p signifie les nombres entiers zéro, 1 ou 2,
7. -$NO_2$,
8. -CN ou
9. -N(R3)-R4, où R3 signifie

   9.1) un atome d'hydrogène ou
   9.2) -($C_1$-$C_6$)-alkyle et R4 est défini comme ci-dessus,

X représente -OH ou -NH-OH,
$X_1$ et $X_2$ sont identiques ou différents et représentent, indépendamment l'un de l'autre, un atome d'hydrogène ou -($C_1$-$C_6$)-alkyle ou forment ensemble le radical =O,
n1 et n2 sont identiques ou différents et représentent, indépendamment l'un de l'autre, zéro, 1 ou 2,

R1 représente

1. un atome d'hydrogène ou
2. -($C_1$-$C_6$)-alkyle, où alkyle est non substitué ou monosubstitué ou disubstitué par -($C_3$-$C_6$)-cycloalkyle, -($C_6$-$C_{14}$)-aryle ou hétéroaryle,

R2 et R3 sont identiques ou différents et représentent, indépendamment l'un de l'autre, un atome d'hydrogène ou -($C_1$-$C_6$)-alkyle,

les composés acide 1-(toluène-4-sulfonyl)-octahydroindole-2-carboxylique et acide N-[(3,5-dichlorobenzène)-sulfonyl)]-(3a(S),7a(S))-octahydroindole-2(S)-carboxylique étant exclus.

**2.** Composé de formule I selon la revendication 1, où

A représente -($C_0$-$C_4$)-alkylène,
B, D et E sont identiques ou différents et représentent, indépendamment l'un de l'autre,

1. -($C_0$-$C_4$)-alkylène,
2. -($C_2$-$C_4$)-alcénylène,
3. -$S(O)_o$-, où o signifie les valeurs entières zéro, 1 ou 2,
4. -NH-,
5. -NH-C(O)-,
6. -C(O)-NH-,
7. -NH-$SO_2$-,
8. -NH-C(O)-NH-,
9. -NH-C(S)-,
10. -NH-C(O)-O-,
11. -O-,
12. -O-C(O)-NH-,
13. -C(O)-,
14. -O-$(CH_2)_n$-O-, où n signifie le nombre entier 2 ou 3, ou
15. -O-$(CH_2)_m$-NH-, où m signifie le nombre entier 2 ou 3,

cycle1, cycle2 ou cycle3 sont identiques ou différents et représentent, indépendamment l'un de l'autre,

1. une liaison covalente,
2. -($C_6$-$C_{14}$)-aryle, où aryle signifie un radical de la série phényle, naphtyle, 1-naphtyle, 2-naphtyle, biphénylyle, 2-biphénylyle, 3-biphénylyle, 4-biphénylyle, anthryle ou fluorényle et sont non substitués ou monosubstitués, disubstitués ou trisubstitués, indépendamment l'un de l'autre, par G, ou
3. un cycle hétéroaryle aromatique de 5 ou 6 chaînons, où le cycle hétéroaryle signifie un radical de la série dihydrofurannyle, dioxolyle, dioxanyle, furannyle, imidazolidinyle, imidazolinyle, imidazolyle, isoxazolyle, isoxazolidinyle, 2-isoxazolinyle, isothiazolyle, isothiazolidinyle, 2-isothiazolinyle, morpholinyle, oxazolyle, oxothiolanyle, pipérazinyle, pipéridinyle, pyrannyle, pyrazinyle, pyrazolyle, pyrazolidinyle, pyrazolinyle, pyridazinyle, pyridinyle, pyrimidinyle, pyrrolyle, pyrrolidinyle, tétrahydrofurannyle, tétrahydropyridinyle, thiazolyle, thiomorpholinyle, thiophényle ou thiopyrannyle et sont non substitués ou monosubstitués, disubstitués ou trisubstitués, indépendamment l'un de l'autre, par G,

cycle4 représente

1. -($C_6$-$C_{14}$)-aryle, où aryle signifie un radical de la série phényle, naphtyle, 1-naphtyle, 2-naphtyle, biphénylyle, 2-biphénylyle, 3-biphénylyle, 4-biphénylyle, anthryle ou fluorényle et est non substitué ou monosubstitué, disubstitué ou trisubstitué, indépendamment l'un de l'autre, par G,
2. un cycle hétéroaryle aromatique de 5 ou 6 chaînons, où le cycle hétéroaryle signifie un radical de la série dihydrofurannyle, dioxolyle, dioxanyle, furannyle, imidazolidinyle, imidazolinyle, imidazolyle, isoxazolyle, isoxazolidinyle, 2-isoxazolinyle, isothiazolyle, isothiazolidinyle, 2-isothiazolinyle, morpholinyle, oxazolyle, oxothiolanyle, pipérazinyle, pipéridinyle, pyrannyle, pyrazinyle, pyrazolyle, pyrazolidinyle, pyrazolinyle, pyridazinyle, pyridinyle, pyrimidinyle, pyrrolyle, pyrrolidinyle, tétrahydrofurannyle, tétrahydropyridinyle, thiazolyle, thiomorpholinyle, thiophényle ou thiopyrannyle et est non substitué ou monosubstitué, disubstitué

ou trisubstitué, indépendamment l'un de l'autre, par G,
3. représente hétéroaryle, où hétéroaryle représente un radical de la série acridinyle, azétidinyle, benzimidazolyle, benzofurannyle, benzothiofurannyle, benzothiophényle, benzoxazolyle, benzothiazolyle, benzotriazolyle, benzotétrazolyle, benzoisoxazolyle, benzo-isothiazolyle, benzoimidazalinyle, carbazolyle, 4aH-carbazolyle, carbolinyle, chromanyle, chroményle, cinnolinyle, décahydroquinoléinyle, 2H,6H-1,5,2-dithiazinyle, dihydrofuranne[2,3-b]-tétrahydrofurannyle, furannyle, furazanyle, imidazolidinyle, imidazolinyle, imidazolyle, 1H-indazolyle, indolinyle, indolizinyle, indolyle, 3H-indolyle, isobenzofurannyle, isochromanyle, iso-indazolyle, iso-indolinyle, iso-indolyle, isoquinoléinyle (benzo-imidazolyle), isothiazolyle, isoxazolyle, morpholinyle, naphtyridinyle, octahydro-isoquinoléinyle, oxadiazolyle, 1,2,3-oxadiazolyle, 1,2,4-oxadiazolyle, 1,2,5-oxadiazolyle, 1,3,4-oxadiazolyle, oxazolidinyle, oxazolyle, oxazolidinyle, pyrimidinyle, phénanthridinyle, phénanthrolinyle, phénazinyle, phénothiazinyle, phénoxathiinyle, phénoxazinyle, phtalazinyle, pipérazinyle, pipéridinyle, ptéridinyle, purynyle, pyrannyle, pyrazinyle, pyrazolidinyle, pyrazolinyle, pyrazolyle, pyridazinyle, pyrido-oxazolyle, pyrido-imidazolyle, pyridothiazolyle, pyridothiophényle, pyridinyle, pyridyle, pyrimidinyle, pyrrolidinyle, pyrrolinyle, 2H-pyrrolyle, pyrrolyle, quinazolinyle, guinoléinyle, 4H-quinoléizinyle, quinoxalinyle, quinuclidinyle, tétrahydrofurannyle, tétrahydro-isoquinoléinyle, tétrahydroquinoléinyle, 6H-1,2,5-thiadazinyle, 1,2,3-thiadiazolyle, 1,2,4-thiadiazolyle, 1,2,5-thiadiazolyle, 1,3,4-thiadiazolyle, thianthrényle, thiazolyle, thiényle, thiénothiazolyle, thiéno-oxazolyle, thiéno-imidazolyle, thiophényle, triazinyle, 1,2,3-triazolyle, 1,2,3-triazolyle, 1,2,4-triazolyle, 1,2,5-triazolyle, 1,3,4-triazolyle et xanthényle et est non substitué ou monosubstitué, disubstitué ou trisubstitué, indépendamment l'un de l'autre, par G, ou
4. un des radicaux suivants

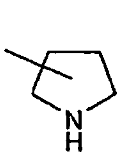
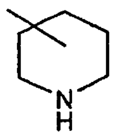
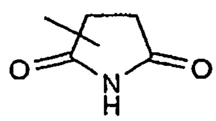
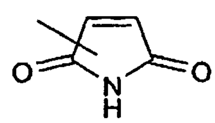
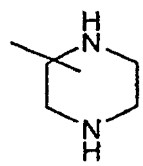
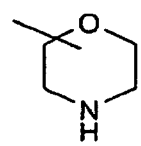

et ces radicaux sont non substitués ou monosubstitués, disubstitués ou trisubstitués, indépendamment l'un de l'autre, par G,

G représente

1. un atome d'hydrogène
2. halogène
3. R4 et R4 signifie

a) un atome d'hydrogène
b) $-(C_1-C_6)$-alkyle, où alkyle est non substitué ou monosubstitué, disubstitué ou trisubstitué par halogène, $-(C_3-C_6)$-cycloalkyle, phényle, naphtyle ou hétéroaryle, où hétéroaryle est tel que défini ci-dessus,
c) phényle ou naphtyle,
d) hétéroaryle, où hétéroaryle est défini comme ci-dessus,
e) -C(O)-O-R5, où R5 signifie

e)1) $-(C_1-C_6)$-alkyle, où alkyle est non substitué ou monosubstitué ou disubstitué par $-(C_3-C_6)$-cycloalkyle, phényle, naphtyle ou hétéroaryle, où hétéroaryle est tel que défini ci-dessus,
e)2) phényle ou naphtyle ou
e)3) hétéroaryle, où hétéroaryle est substitué comme défini comme ci-dessus,

f) -C(S)-O-R5, où R5 est défini comme ci-dessus,
g) -C(O)-NH-R6, où R6 signifie

g)1) $-(C_1-C_6)$-alkyle, où alkyle est non substitué ou monosubstitué ou disubstitué par $-(C_3-C_6)$-cycloalkyle, phényle, naphtyle ou hétéroaryle, où hétéroaryle est tel que défini ci-dessus,
g)2) phényle ou naphtyle ou
g)3) hétéroaryle, où hétéroaryle est substitué comme défini comme ci-dessus, ou

h) -C(S)-NH-R6, où R6 est défini comme ci-dessus,

4. -O-R4, où R4 est défini comme ci-dessus,
5. -C(O)-R5, où R5 est défini comme ci-dessus,
6. $-S(O)_p$-R4, où R4 est défini comme ci-dessus et p signifie les nombres entiers zéro, 1 ou 2,
7. $-NO_2$,
8. -CN ou
9. -N(R3)-R4, où R3 signifie

9.1) un atome d'hydrogène ou
9.2) $-(C_1-C_6)$-alkyle et R4 est défini comme ci-dessus,

X représente -OH ou -NH-OH,
X1 et X2 sont identiques ou différents et représentent, indépendamment l'un de l'autre, un atome d'hydrogène ou $-(C_1-C_6)$-alkyle ou forment ensemble le radical =O,
n1 et n2 sont identiques ou différents et représentent, indépendamment l'un de l'autre, zéro, 1 ou 2,
R1 représente

1. un atome d'hydrogène ou
2. $-(C_1-C_6)$-alkyle, où alkyle est non substitué ou monosubstitué ou disubstitué par $-(C_3-C_6)$-cycloalkyle, phényle, naphtyle ou hétéroaryle, où hétéroaryle est tel que défini ci-dessus,

R2 et R3 sont identiques ou différents et représentent, indépendamment l'un de l'autre, un atome d'hydrogène ou $-(C_1-C_6)$-alkyle.

**3.** Composé de formule I selon les revendications 1 ou 2, où

A représente $-(C_0-C_4)$-alkylène,
B, D et E sont identiques ou différents et représentent, indépendamment l'un de l'autre,

1. $-(C_0-C_2)$-alkylène,
2. $-C_2$-alcénylène,
3. $-S(O)_o$-, où o signifie le nombre entier 2,
4. -NH-,
5. -NH-C(O)-,
6. -C(O)-NH-,
7. -NH-C(O)-NH-,
8. -O- ou
9. -C(O)-,

cycle1, cycle2 et cycle3 sont identiques ou différents et représentent, indépendamment l'un de l'autre,

1. une liaison covalente,
2. phényle ou naphtyle et sont non substitués ou monosubstitués, disubstitués ou trisubstitués, indépendamment l'un de l'autre, par G,
3. un cycle hétéroaryle aromatique de 5 ou 6 chaînons, où le cycle hétéroaryle signifie un radical de la série dihydrofurannyle, furannyle, morpholinyle, pipérazinyle, pipéridinyle, pyridinyle, pyrimidinyle, pyrrolyle, thiazolyle ou thiophényle et sont non substitués ou monosubstitués, disubstitués ou trisubstitués, indépendamment l'un de l'autre, par G,

cycle4 représente

1. phényle ou naphtyle et est non substitué ou monosubstitué, disubstitué ou trisubstitué, indépendamment l'un de l'autre, par G,
2. un cycle hétéroaryle aromatique de 5 ou 6 chaînons, où le cycle hétéroaryle signifie un radical de la série dihydrofurannyle, furannyle, morpholinyle, pipérazinyle, pipéridinyle, pyridinyle, pyrimidinyle, pyrrolyle, thiazolyle, thiophényle et est non substitué ou monosubstitué, disubstitué ou trisubstitué, indépendamment l'un de l'autre, par G,
3. hétéroaryle où hétéroaryle signifie un radical de la série benzofurannyle, benzothiophényle, dihydrofurannyle, furannyle, morpholinyle, pipérazinyle, pipéridinyle, pyridinyle, pyrothiophényle, pyrimidinyle, pyrrolyle, thiazolyle ou thiophényle et est non substitué ou monosubstitué, disubstitué ou trisubstitué, indépendamment l'un de l'autre, par G,

4. un des radicaux suivants

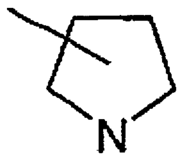

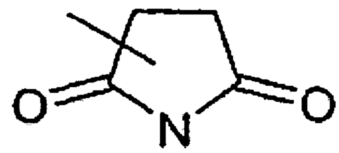

et ces radicaux sont non substitués ou monosubstitués, disubstitués ou trisubstitués, indépendamment l'un de l'autre, par G,

G représente

1. un atome d'hydrogène
2. Br, Cl, I ou F,
3. R4 et R4 signifie

a) un atome d'hydrogène
b) -($C_1$-$C_4$)-alkyle, où alkyle est non substitué ou monosubstitué, disubstitué ou trisubstitué par Br, Cl, F, -$C_3$-cycloalkyle, phényle, naphtyle ou hétéroaryle, où hétéroaryle est défini comme ci-dessus,
c) phényle ou naphtyle,
d) hétéroaryle, où hétéroaryle est défini comme ci-dessus,
e) -C(O)-O-R5, où R5 signifie

e)1) -($C_1$-$C_4$)-alkyle, où alkyle est non substitué ou monosubstitué ou disubstitué par -$C_3$-cycloalkyle, phényle, naphtyle ou hétéroaryle, où hétéroaryle est défini comme ci-dessus,
e)2) phényle ou naphtyle ou
e)3) hétéroaryle, où hétéroaryle est substitué comme défini comme ci-dessus, ou

f) -C(O)-NH-R6, où R6 représente

f)1) -($C_1$-$C_4$)-alkyle, où alkyle est non substitué ou monosubstitué ou disubstitué par -C3-cycloalkyle, phényle, naphtyle ou hétéroaryle, où hétéroaryle est défini comme ci-dessus,
f)2) phényle ou naphtyle ou
f)3) hétéroaryle, où hétéroaryle est substitué comme défini ci-dessus,

4. -O-R4, où R4 est défini comme ci-dessus,
5. -C(O)-R5, où R5 est défini comme ci-dessus,
6. -$S(O)_p$-R4, où R4 est défini comme ci-dessus et p signifie le nombre entier 2,
7. -$NO_2$,
8. -CN ou
9. -N(R3)-R4, où R3 signifie

9.1) un atome d'hydrogène ou
9.2) -($C_1$-$C_6$)-alkyle et R4 est défini comme ci-dessus,

X représente -OH ou -NH-OH,
$X_1$ et $X_2$ sont identiques et signifient un atome d'hydrogène,
n1 et n2 sont identiques et signifient 1, ou sont différents et n1 signifie 2 et n2 signifie 1,
R1 représente un atome d'hydrogène, et
R2 et R3 sont identiques et signifient un atome d'hydrogène.

**4.** Procédé pour la préparation du composé de formule I selon l'une ou plusieurs des revendications 1 à 3 et/ou d'une forme stéréo-isomère du composé de formule I et/ou d'un sel physiologiquement acceptable du composé de formule

I qui est **caractérisé en ce qu'**on transforme

a) un composé de formule IV,

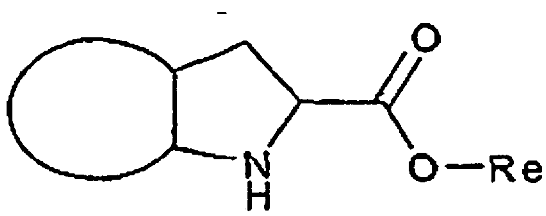

(IV)

où Re représente un atome d'hydrogène ou un groupe de protection de la fonction ester, avec un composé de formule V,
où A, B, D, E et cycle1, cycle2, cycle3, cycle4 sont définis comme dans la formule I et où Rz signifie un atome de chlore, imidazoyle ou OH,

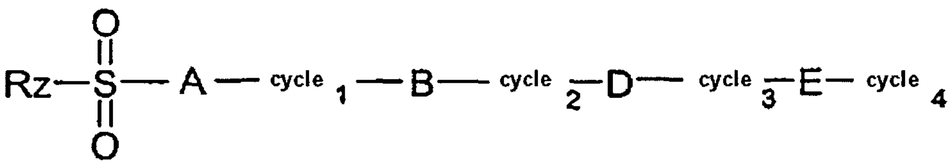

(V)

en présence d'une base ou après silylation avec un agent de silylation approprié en un composé de formule VI,

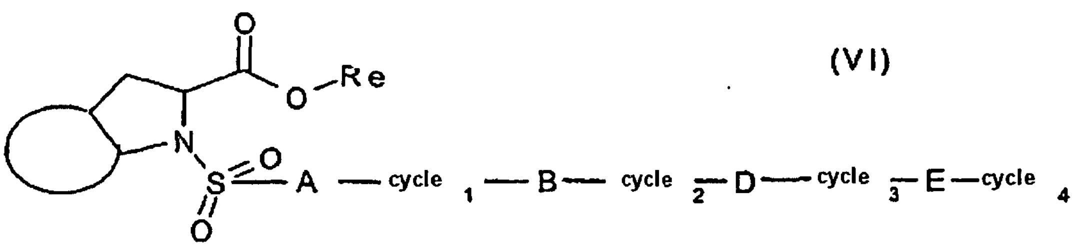

(VI)

où A, B, D, E, Re et cyclel, cycle2, cycle3, cycle4 sont définis comme ci-dessus,
b) dans le cas où Re = ester, on transforme un composé de formule VI préparé selon a) avec une lessive alcaline telle que NaOH ou LiOH et par un traitement acide consécutif en acide carboxylique selon l'invention de formule I, en réalisant le cas échéant encore au préalable des modifications dans l'une des chaînes latérales des cycles cyclel-cycle4

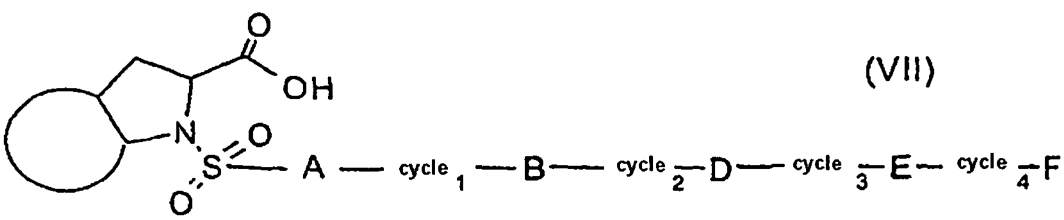

(VII)

et on transforme ensuite celui-ci en acide hydroxamique selon l'invention, où X = NH-OH, de formule I,
c) on sépare un composé de formule I, préparé selon le procédé a) ou b), qui, en raison de sa structure chimique, apparaît sous des formes énantiomères, par salification avec des acides ou des bases sous forme d'énantiomères purs, chromatographie sur des phases stationnaires chirales ou transformation en dérivés au moyen de composés sous forme d'énantiomères purs, tels que des aminoacides, séparation des diastéréo-isomères ainsi

obtenus et élimination des groupes auxiliaires chiraux, en énantiomères purs, ou
d) le composé de formule I, préparé selon les procédés b) ou c) est soit isolé sous forme libre, soit, dans le cas de la présence de groupes acides ou basiques, converti en sels physiologiquement acceptables.

**5.** Médicament, **caractérisé par** une teneur active en au moins un composé de formule I selon l'une ou plusieurs des revendications 1 à 3 avec un support, un additif et/ou d'autres substances actives et adjuvants pharmaceutiquement approprié(s) et physiologiquement compatible(s).

**6.** Utilisation du composé de formule I selon l'une ou plusieurs des revendications 1 à 3 pour la préparation d'un médicament pour la prophylaxie et la thérapie de maladies telles que les ostéoarthroses, les spondyloses, une atrophie des cartilages après un traumatisme articulaire ou un repos prolongé des articulations après des lésions au ménisque ou à la rotule ou des déchirures de ligaments, les maladies du tissu conjonctif, telles que les collagénoses, les maladies du périodonte, les troubles de guérison de plaies et les maladies chroniques de l'appareil locomoteur, telles que les arthrites aiguës et chroniques, inflammatoires, immunologiques ou dues au métabolisme, les arthropathies, les myalgies et les troubles du métabolisme des os, pour le traitement de l'ulcération, de l'athérosclérose et des sténoses, le traitement d'inflammations, de maladies cancéreuses, la formation de métastases tumorales, la cachexie, l'anorexie, l'insuffisance cardiaque et le choc septique ou la prophylaxie de l'infarctus du myocarde et cérébral.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0606046 A
- WO 9428889 A
- WO 9627583 A
- WO 9718194 A


### In der Beschreibung aufgeführte Nicht-Patentliteratur


- **D. Yip et al.** *Investigational New Drugs,* 1999, vol. 17, 387-399
- **Michaelides et al.** *Current Pharmaceutical Design,* 1999, vol. 5, 787-819
- **S. J. George.** *Exp. Opin. Invest. Drugs,* 2000, vol. 9 (5), 993-1007
- **E. J. M. Creemers et al.** *Circulation Res.,* 2001, vol. 89, 201-210
- *Drugs,* 2001, vol. 61, 1239-1252
- *Current Medicinal Chemistry,* 2001, vol. 8, 425-74
- **Massova I et al.** *The FASEB Journal,* 1998, vol. 12, 1075-1095
- *Tetrahedron,* vol. 47 (18-19), 3077
- **T.H. Greene ; P. G. M. Wuts.** Protective Groups in Organic Synthesis. Wiley-Interscience, 1991
- **Ye et al.** *Biochemistry,* 1992, vol. 31, 11231-11235